# EUROPÄISCHE PATENTSCHRIFT

(11) **EP 2 395 973 B1**
(45) Veröffentlichungstag und Bekanntmachung des Hinweises auf die Patenterteilung: **03.04.2013**
(21) Anmeldenummer: 10703002.5
(22) Anmeldetag: 27.01.2010
(51) Int. Cl.: A61K 9/06, A61K 9/107

(54) **IN SITU HAFT-GELBILDENDE ZUBEREITUNGEN, INSBESONDERE ZUR TOPISCHEN ANWENDUNG AUF BEFEUCHTETE HAUT/SCHLEIMHAUT**
IN SITU PREPARATIONS FORMING BONDING GEL, IN PARTICULAR FOR TOPICAL APPLICATION TO MOISTENED SKIN/MUCOUS MEMBRANES
PRÉPARATIONS FORMANT IN SITU UN GEL ADHÉSIF, EN PARTICULIER POUR APPLICATION TOPIQUE SUR UNE PEAU/UNE MUQUEUSE HUMIDIFIÉE

(30) Priorität: 09.02.2009 DE 102009008094
(43) Veröffentlichungstag der Anmeldung: 21.12.2011
(73) Patentinhaber: Barnikol-Keuten, Doris, 55128 Mainz (DE)
(72) Erfinder: Barnikol-Keuten, Doris, 55128 Mainz (DE)
(74) Vertreter: Müller, Claudia
(86) Internationale Anmeldenummer: PCT/EP2010/000481
(87) Internationale Veröffentlichungsnummer: WO 2010/089046

(56) Entgegenhaltungen:
- WO-A1-95/14037
- WO-A2-2004/017998
- WO-A2-2005/105040
- US-A1- 2003 083 314
- US-A1- 2007 259 013

## Beschreibung

### Gegenstand der Erfindung

Die vorliegende Erfindung betrifft lipid- und wasserhaltige Zubereitungen mit einer besonderen Kombination aus nicht ionischen niedrig(poly)ethoxylierten O/W-Emulgatoren mit einer oder mehreren Ether- oder Esterbindungen mit insbesondere 2-, 3- oder 4-wertigen (Poly)Alkoholen einschließlich Sorbitan sowie höher ethoxylierten nicht ionischen Gel- Emulgatoren. Derartige Zubereitungen bilden bei Anwendung auf insbesondere Schleimhäuten in situ "irreversible" (permanent-) Gele aus und weisen insofern ein verbessertes Haft- und Wirkungsspektrum auf. Die erfindungsgemäßen Zubereitungen sind daher geeignet für die topische Anwendung auf äußerlich erreichbare, insbesondere befeuchtete Haut, vor allem Schleimhäute bei Mensch und Tier, wie im Mund- und Nasenraum, der Scheide, Harnröhre sowie Analbereich, oder auch die Bindehaut des Auges. Dabei kann es sich um eine kosmetisch- dermatologische oder um eine pharmazeutisch medizinische Anwendung handeln.

### Hintergrund der Erfindung

Die Schleimhäute von Menschen und Tieren werden häufig infektiös befallen und zeigen als Folge entzündliche, teils schmerzhafte Reaktionen, wie im Fall des Herpes. Im Mund / Halsbereich sind es vorwiegend Erkältungskrankheiten, aber auch der Befall des Zahnapparates durch die kariogene Mikroflora, begleitet von Parodontopathien wie Gingitividen. In der Scheide findet sich häufig eine mykogene Vaginitis. Die Urethra ist meistens bakteriell befallen und im Analbereich stellen sich oft schmerzhaft-entzündliche Beschwerden auf Grund einer Varizenbildung ein Auch die äußere Haut kann durch Infektionen /Entzündungen aus dem Gleichgewicht geraten.

Allermeistens ist es nicht indiziert, solche lokalen Störungen systemisch zu behandeln. Daher verbleibt nur eine topische Therapie, wobei es entscheidend darauf ankommt, dass die Wirkstoffe lange, am besten über Tage, lokal vorgehalten werden. Zu diesem Zweck wurden herkömmlicherweise galenische Zubereitungen in Form von Salben, Cremes, Gelen sowie alkoholisch-wässerige Spül-Lösungen entwickelt, welche entsprechende Wirkstoffe enthalten. Jedoch haben diese Zubereitungen den Nachteil, dass sie durch die ständige Sekret-Bildung der Schleimhäute bzw. Schweißbildung der Haut verdünnt und weggeschwemmt werden und somit nicht haften bleiben, sodass die Wirkstoffe lokal nicht lange genug vorgehalten werden können, um die pathologischen Störungen beseitigen zu können. Daher wäre in diesen Fällen insbesondere eine gelartige Formulierung von Vorteil, um durch Adhäsion eine verbesserte Wirksamkeit zu erzielen. Im Falle z. B. des Mundbereiches wäre es beispielsweise wünschenswert, die Schleimhaut / Zähne mit einer festen adhäsiven Schicht zu überziehen und gleichsam irreversibel, vor allem über einen längeren Zeitraum wie über Nacht bestehen zu lassen, so dass die Wirkstoffe sich besser entfallen könnten.

### Problematik

Aufgrund der Beschaffenheit der Schleimhäute bzw. der äußeren Haut müssen hierfür geeignete Zubereitungen wie Gele, welche haften sollen, hydrophil sein. Ein besonderes Problem der Schleimhaut besteht dabei darin, dass sie sezemiert, so dass ein herkömmliches "passives" Gel schnell verdünnt, wegschwemmt wird und dadurch uneffektiv wird. Die natürliche Ausdünstung der oberen Hautschicht (Feuchtigkeit /Schweiß u. ä.) kann ebenfalls zur Fluidisierung von Gelen führen. Darüber hinaus sind viele Wirkstoffe lipophil oder lipophil-hydrophil (also amphiphil). wodurch das Problem der ausreichenden molekular- dispersen Einarbeitung dieser Wirksubstanzen in die Zubereitungen entsteht. Letzteres Problem kann zufriedenstellend mit Mikro-Emulsionen gelöst werden. Diese bestehen prinzipiell aus Öl, Tensid und Co-Tensid, sowie aus Wasser. Sie können lipophile Wirkstoffe in der Ölphase, hydrophile in der wässerigen Phase und amphiphile Wirkstoffe in der extrem großen inneren Phasen-Grenzfläche molekular-dispers aufnehmen. Mikro-Emulsionen können insofern quasi als "Lösungsmittel", d.h. molekulare Dispersions-Systeme Wirksubstanzen eingesetzt werden. Allerdings haften Mikro- Emulsionen nicht ohne weiteres auf Schleimhäuten. Vielmehr vermögen Mikro-Emulsion alleine nur reversible gelige nicht haftende Fluide zu bilden, weil sie aus mehr-dimensionalen Flüssigstrukturen aus Bilipid-Schichten in Form von Tubuli, Lamellen und Kuboiden bestehen, die sich bei Konvertierung der Emulsion durch Flüssigkeits- /Feuchtigkeitszusatz wieder zu einem Fluid mit O/W-Mizellen umordnen.

### Stand der Technik

Es hat nicht an Versuchen gefehlt, entsprechend leistungsfähige Gele zu entwickeln und herzustellen:
Gemäß der US 5200393 wird eine Zubereitung für Peptide wie z. B. Insulin zur intranasalen / transmukosalen, also systemischen Anwendung vorgeschlagen, welche zur Gel-Bildung ein Oleyl-Phospholipid aufweist, das bei 1-2%iger Konzentration in Wasser ein Gel bildet, allerdings beim Verdünnen auf 0,8% schon dünnflüssig wird und nicht mehr haftet. Zudem muss für die Gelbildung der pH-Wert auf 9 eingestellt bleiben. Für Schleimhäute ist ein derartiger Wert jedoch ungeeignet, da deren eigener pH-Wert deutlich niedriger liegt.

Nach der ES 2095183 wird ebenfalls eine intränasale Applikation angestrebt, um Wirkstoffe, insbesondere Sympathomimetika, systemisch anzuwenden. Hier handelt es sich um ein klassisches hydrophiles Gel mit etwa 10% Polaxomer sowie als weitere synthetische Gel-Bildner Carboxypolymethylen oder Hydroxypropyl-Cellulose oder Hydroxypropylmethyl-Cellulose oder Derivate der Poly-Acrylsäure. Allerdings verliert das Gel bei Verdünnung durch Sekret-Bildung seine Konsistenz. In der US 6596763 werden herkömmliche Hydrogele beschrieben, die mindestens 1 microbicidales Lipid (C6-C18-Fettsäuren), mindestens 1 Lösungs-Agenz (Glycofurol) und optional einen Gel-Bildner, wie Carbopol, enthalten. Auch diese Gele verliehen durch die Schleimhaut-Sekretion schnell ihre Konsistenz und Haftung. Gemäß der RO 121726 wird eine einfache gelige Zubereitung hauptsächlich auf Basis von Polycarboxylsäuren, Flavonen, Palmitinsäure und ungesättigter Palmitinsäure hergestellt, ohne dass es eine Konsistenz-Resistenz gäbe.

In CN1887943 wird ein Gel-Film beschrieben, der aus synthetischen Substanzen, wie Hydroxy-Alkyl-Cellulose, Vemetzem und Ester-Bildnem in einem geeigneten Lösungsmittel entsteht. Dieser hydrophobe Film kann insbesondere lipophile Wirkstoffe aufnehmen und an der oralen Mukosa angewendet werden. Es handelt sich hier um eine Art Lacküberzug und nicht um ein Gel.

In CN1579552 wird über ein aus Polymeren aufgebautes Micell-System berichtet (unter Nutzung der sogen. "glue beam technology"), mit welchem die verschiedenen Wirkstoffe, auch Polypeptide, durch die Haut und Schleimhaut transportiert werden können.

Die WO 0149276 A2 betrifft ein wasserfreies Gel, welches u.a. aufgebaut ist aus Polyethylen-Glykol verschiedenen Molekulargewichts sowie dessen Mono-ethylEther, ferner aus Poly-Acrylsäure. Lipophile Wirkstoffe wie nicht steroidale Entzündungshemmer, besonders Diclofenac, können in das Gel eingearbeitet werden, das zur anti-entzündlichen systemischen Therapie, vor allem an der Schleimhaut des Mundbereichs, angewendet werden soll.

In WO 2007018622 A2 wird ein hydrophiles Gel beschrieben, welches sich nach der Anwendung durch die Gegenwart 1-und 2-wertiger Ionen bildet, die sich in dem betreffenden Schleimhaut-Sekret befinden müssen. Gel-Bildner sind anionische Polysaccharide. Hier ist insbesondere eine vaginale Anwendung vorgesehen.

In WO2007144139 A2 werden Doppel-Mikro-Emulsionen (W/O/W und O/W/O) beschrieben, in die gewünschte Effektoren eingebracht werden können, mit dem Ziel einer intravaginalen lokalen Therapie.

### Aufgabe vorliegender Erfindung

Keine der o.g. Zubereitungen ist in der Lage, allen gewünschten Erfordernissen einer erfolgreichen lokalen topischen, insbesondere Schleimhaut-Therapie zugleich gerecht zu werden, nämlich:
a) ein lange (über Stunden) haftendes Gel zu bilden und die Applikationsstellen gleichsam zu versiegeln, wobei es jedoch
b) keiner Entfernung der Zubereitung bedarf;
c) hohe Konzentrationen lipophiler, hydrophiler sowie insbesondere amphiphiler Wirkstoffe möglichst lange Zeit (über Stunden) lokal vorzuhalten;
d) eine gute bzw. verstärkte Penetration der Wirkstoffe in die Haut, vor allem Schleimhaut zu ermöglichen.

### Lösung der Aufgabe und kurze Charakterisierung der Erfindung

Diese Aufgabe wird erfindungsgemäß gelöst durch Zubereitungen, vor allem in Form von W/O- Mikro-Emulsionen aus einer Ölphase (30 - 80 Gew.%), insbesondere einer flüssigen Ölphase, wie z.B. einer flüssigen Phase aus einem oder mehreren Lipoiden, und einer Wasserphase, welche weiterhin eine Kombination aus (einem oder mehreren) nicht ionischen niedrig (poly)ethoxylierten Emulsions-Emulgatoren mit einer oder mehreren Ether- / und / oder Esterbindungen mit insbesondere C2-Di- C3 -Trialkoholen oder C4-Tetra-, oder C6-Hexa-(Poly)Alkoholen (auch Sorbitanen) und (einem oder mehreren) höher oxylierten, insbesondere ethoxylierten - nicht ionischen Gel- Emulgatoren mit einem hydrophilen Teil aus linearen / oder verzeigten PEG- (= Polethylenglykol)- Ketten aufweisen. Überraschenderweise wurde gefunden, dass sich bei Kontakt derartiger Zubereitungen mit dem wässrigen Sekret der Haut wie Schweiß oder auch Sekret, insbesondere mit dem wässerigen Sekret der Schleimhäute, in situ ein lange haftendes "irreversibles" Permanent- Gel bildet, ohne dass spezielle, vor allem synthetische Gelbildner wie vorstehend genannt (z.B. Acrylate, Cellulosederivate) dazu erforderlich wären, was von analogen Mikro-Emulsions-Fluiden nicht erwartet werden konnte. Somit kann die Mikro-Emulsion als effektives Dispersionssystem für die Wirkstoffe genutzt werden, das unerwarteter Weise ein Anwendungsgel mit guter Haftung ausbildet. Dabei können auch thixotrope Flüssig-Strukturen entstehen, welche die Gel-Verfestigung und damit die Haftung weiter verbessern.

### Nähere Erläuterung der Erfindung

Die Erfindung betrifft insofern Zubereitungen (insbesondere W/O), enthaltend:
- 30 bis 80 Gew.% einer flüssigen Ölphase, aus einem oder mehreren Lipoiden;
- 5 bis 45 Gew.% einer Mischung aus:
   a) einem oder mehreren höher (eth)oxylierten Gel- Emulgatoren der allgemeinen Formel **I**

      R₁(m₁) - An -R₂ (ₘ₂). (I)

      worin R₁, R₂ ausgewählt sind aus H, C₁₂ - C₂₂, gesättigten, und / oder ungesättigten Fettsäureglycerid-Ölen, insbesondere essentiellen und / oder hydroxysubstituierten Ölen; C₁₂ - C₂₂ Fettalkoholen, C₁₂ - C₂₂, -Fettsäuren, oder Sorbitan- C₁₂-C₂₀ Fettsäureestem, oder (ggf.oligo /poly) Erythrol-Fettsäure-Estern, wobei A= (O CH₂ -CH₂-), und n = 60 bis 1000 und m₁ = 0 bis 2, m₂ =1 bis 2 bedeuten, mit der Maßgabe, dass die Summe aus m, + m₂ (mit m₂ ≠H) ≤ 2 ist; wobei 0,005 bis 20 Gew.% des Gel- Emulgators a) in der Mischung aus a) +b), bevorzugt 0,005 bis 13 Gew.%, vor allem 0,005 bis 8 Gew.%, vorhanden sind; und
   b) einem oder mehreren nicht ionischen Emulsions- Emulgatoren der allgemeinen Formel II

      (R₂)ₓ₁ - (A)_{y} -(R₁)ₓ₂ (II),

      worin A= (O - CH₂ -CH₂-), (O - CH₂ -C (CH₃)H-) oder (O - B-R₂ₓ₁) bedeutet; R₁ ausgewählt ist aus gesättigten und oder ungesättigten C₁₂ - C₂₂, Fettsäureglycerid-Ölen, C₁₂ - C_{22'} - Fettalkohlen, - C₁₂ - C_{22'} - Fettsäuren, Sorbitan C₁₂-C₂₀-Fettsäure-estern; Sterolen, besonders natürlicher Herkunft; R₂ gewählt wird aus gesättigten, und oder ungesättigten - C₁₂ - C₂₂- Fettalkoholen, gesät tigten und /oder ungesättigten C₁₂ - C₂₂- Fettsäureglyocrid-Ölen, - C₁₂ - C_{22'} - Fettsäuren; H; mit y= 1-50, vorzugsweise 2-50, und mit x₁= 0 bis 10 und ,x₂ = 1 bis 5, wobei die Summe x, + x₂ (R_{1,2} # H) 1-10, vorzugsweise 1-6, beträgt; B (mono- oder poly)-Di- bis hexahydroxy- C₂ bis C₆- Alkohol, vorzugsweise (mono-oder poly)- Ethylenglykol, -Glycerol, - Sucrose, -Erythrol oder - Sorbitol bedeutet;
- 0 bis 20 Gew.% eines oder mehrerer 1- und / oder 2- wertiger C₂-C₈- Alkohole;
- 1 bis 15 Gew.% Wasser oder wässerige Lösungen.

Weiterhin sind je nach Indikation geeignete lipophile, hydrophile sowie amphiphile Wirkstoffe insgesamt zwischen 0 und 40 Gew.%, bevorzugt zwischen 0,001 und 35 Gew.% vorhanden.

Schließlich sind Zusatzstoffe möglich, wie Penetrations- und Diffusionsverstärker, Anti-Oxidantien (insbesondere zur Stabilisation von Doppelbindungen der Komponenten), sowie Salze, niedermolekulare Säuren, Laugen (jeweils als pH-Wert-Regulatoren), die im Allgemeinen insgesamt in einer Menge von 0,0 bis 30 Gew.%, bevorzugt zwischen 0,1 und 25 Gew.% vorhanden sein können. Auch können ggf. Geschmacksstoffe, amphotere Tenside und lipophile Wasserbinder zur Stabilisation, ggf. auch Konservierungsstoffe, in hierfür üblichen Mengen enthalten sein.

Die Menge an Öl(phase) (Lipide) beträgt vorzugsweise 35 bis 70 Gew.%, insbesondere 37 bis 65 Ges.%.

Die Ölphase (Lipoidphase) ist vor allem flüssig und wird gemäß vorliegender Erfindung gebildet aus Ölen, insbesondere flüssigen bis wachsartigen Ölen, bzw. Lipoiden oder Mischungen hiervon. Hierzu gehören vor allem:
- Ester aus monovalenten C₃-C₃₀- Alkoholen mit C₆-C₁₈- Carbonsäuren, wie Isopropyl-Palmitat, -Myristat, -Stearat;n-Hexyl-Laurat;
- weiterhin gesättigte oder ungesättigte C₁₂-C₂₀-Fettsäuren natürlicher Herkunft wie Ölsäure, Linolsäure, Palmitinsäure;
- ferner Glycerolester oder C₁₂-C₂₀- Alkylether gesättigter und ungesättigter C₁₂-C₂₀-Fettsäuren natürlicher und synthetischer Herkunft wie vor allem Triglyceride (Carpryl- Caprinsäuretriglycerid),
- weiterhin C₆-C₂₀ Fettalkohole natürlicher oder synthetischer Herkunft wie Lanolin oder Mischungen verschiedener genannter Substanzen.

Besonders bevorzugt sind Isopropyl-Palmitat, Isopropyl-Myristat. - Isopropyl-Stearat, Oleyl-Oleat, Isopropyl-Oleat, Ölsäure, Linolsäure, Linolensäure, Palmitinsäure, Oliven-Öl, Mandel-Öl, Raps-Öl. Avocado-Öl, Sonnenblumen-Öl, Soja-Öl, Erdnuss-Öl, Capryl/caprinsäure-Triglycerid, Nachtkerzensamen-Öl, Borretschsamen-Öl, Johannisbeerkern-Öl, Di-Capryl-Ether, Schwarzkkümmel-Öl, Inophyllum, Calophyllum, Maiskeim-Öl, Jojoba-Öl, Aprikosenkem-Öl, Wildrosen-Öl,Weizenkeim-Öl; oder Lauryl-, Linol-, Linolen-, Arachidon-, Palmitin-Alkohol, Lanolin und acetyliertes Lanolin oder Mischungen hiervon.

Die Gesamtmenge an Emulsionsemulgator(en) und Gel-Emulgator(en) beträgt bevorzugt 15 bis 45%, insbesondere 35 bis 45 Gew.%. In erfindungsgemäßen Emulgatoren bedeuten "C₁₂ - C₂₂-Fettsäureglycerid-Öle" (R_{1,2}) entsprechende (gesättigte, ungesättigte) Mono-, Diglyceride, ggf. substituierte gesättigte oder ungesättigte Triglycerid- (Öl)-Komponenten.

In den beschriebenen Zubereitungen ist bevorzugt ein Überschuss an EmulsionsEmulgator im Verhältnis zum Gel-Emulgator vorhanden, vor allem im Bereich 50: 0,05 bis 50:20, oder auch 50:0,5 bis 50:5.

Bevorzugte Emulsions- Emulgatoren sind ausgewählt aus solchen mit einem HLB-Wert ab 5, vor allem von 7,5 bis 19, (z.B. O/W), insbesondere aus Verbindungen der (Poly)Ethylenglykol- Derivate der Formel (II) mit A= (O- CH₂ -CH₂ -)_{y}, mit R₁. R₂, x₁, x₂ , y, wie angegeben, vor allem aus:
- PEG (2-50)-mono-Estern, PEG (4-50)-di-Estern, PEG(10-40) Glycerolestem oder PEG (1-2)-Fettsäureethem gesättigter und ungesättigter C₁₂-C₂₀ - Fettsäuren bzw. C₁₂-C₂₂-Fettalkoholen;
- PEG(4-50) -Glycerid Ölen mit x₂ + x₂ (R₁, R₂ ≠H) 1 bis 4,.
- PEG(4-50)-Sorbitan- C₁₂-C₂₀ -Fettsäuren (x₂ + x₂ (R₁, R₂ ≠H) =1-6 ; wie insbesondere Polysorbat 80, nämlich PEG(20)- Sorbitanmonooleat;
- PEG(2-50)- C₁₂-C₁₆ Alkylethern;
- PEG(5-30)-Sterolen (= Soja-Sterol, Phyto-Sterol, Cholesterol, Lano-Sterol. Sito-Sterol);
- Sucrose- C₁₂-C₂₂-Fettsäure-mono- und di-Ester, insbesondere Ester mit Stearat, Palmitat, Laurat;
- Propylenglykol-mono-und di-Ester der C₈ -C₂₂-Fettsäuren, wobei Laurat, Myristat, Stearat, Ricinoleat und Oleat bevorzugt sind;
oder Mischungen hiervon.

Als ethoxylierte Gel-Emulgatoren der Formel I sind insbesondere solche mit einem HLB- Wert von 12-19, vor allem 14-19 geeignet, die ausgewählt sind aus:
- PEG (60-1000)-mono-Estern oder PEG (400)- Di-Estern von C₁₄-C₂₀ - Fettsäuren wie PEG(60-1000)- Mono-Oleat, -Stearat, -Laurat, -Ricinolat, PEG (400) di-Oleat, -di-Stearat;
- natürlichen und hydrierten natürlichen PEG- Ölen wie PEG(60 bis 200)-Castoroil, - PEG (60-200)-Castoroil (hydriert), PEG(60-200)-Maisöl, -Olivenöl, - Erdnussöl, -Palmkernöl, -Mandelöl, -Aprikosenkemöl;
- PEG(60; 80)-Sorbitan- C₁₂-C₂₀ -Fettsäurester wie PEG(60; 80)-Sorbitan-Laurat, -Palmitat, -Stearat, -Oleat;
- PEG(60-400)- C₁₄-C₁₈ -Alkyl-Ether wie PEG(60-400)- Oleyl, Stearyl, Lauryl, Cetylether.
oder Mischungen hiervon.

Insbesondere bevorzugt sind PEG60-Mandelöl-Glycerid, PEG90-Aprikosenkemöl-Glycerid, PEG60-Nachtkerzensamen-Glycerid, PEG60-Maiskeimöl-Glycerid, PEG60-Passiflora-Glycerid, PEG100-Stearat

Erfindungsgemäß sind insofern bisher verwendete Gelbildner wie Carboxypolymethylen, Hydroxypropyl-Cellulose, Hydroxypropylmethyl-Cellulose, Carbopol, (Poly)Acrylate oder anionische Polysaccharide nicht erforderlich und bevorzugt nicht enthalten.

Bevorzugt ist der C₁₂-C₂₀ Fettsäure- / Fettalkohol- bzw. C₁₂-C₂₀ -Alkylteil der Emulgatoren abgeleitet von Ölsäure, Linolensäure, Laurinsäure, Linolsäure, Palmitinsäure, Caprinsäure, Ricinolsäure, Stearinsäure Cetylalkohol, Laurylalkohol, Stearylalkohol, Oleylalkohol.

Als Öle (Lipoidphase) kommen vor allem Ester aus monovalenten C₃-C₃₀- Alkoholen mit C₆-C₁₈- Carbonsäuren; gesättigte oder ungesättigte C₁₂-C₂₀-Fetsäuren natürlicher Herkunft; Glycerolester oder C₁₂-C₂₀ Alkylether gesättigter und ungesättigter C,ᵣC₂ₒ-Fettsäuren natürlicher und synthetischer Herkunft; oder Mischungen hiervon in Frage.

Als Wirkstoffe werden vor allem solche ausgewählt aus der Gruppe, umfassend anti-infektiöse (anti-bakterielle, anti-mykotische, anti-septische Wirkstoffe) oder antivirale Wirkstoffe, adstringierende, analgetische, anästesierende, antihämorrhagische, phlebotonische, anti-histaminische, antikoagulierende, anti-ödematöse, anti-phlogistische, antiseptische (relaxierende), regenerierende, wundheilende, pflegende Wirkstoffe, hyperämisierende, immunmodulatosche, juckreizstillende, anti-allergische, vasodilatorische, anti-oxidative, befeuchtende Wirkstoffe sowie hormonal-, sympatisch-, parasympatisch- und Carbo-Anhydrasehemmende Wirkstoffe.

Weiterhin können erfindungsgemäß auch pflegende Wirkstoffe mit amphotherem Charakter wie Phosphatidyl-Derivate wie Phosphatidyl-Cholin (Lecithin), oder Phosphatidyl-Serin eingesetzt werden.

In einer weiteren geeigneten Ausführungsform umfasst die Zubereitung einen oder mehrere ein- und /oder zweiwertige kurzkettige Alkohole wie vor allem Ethanol, Isopropanol oder Mischungen hiervon und / oder Propylenglykol und / oder Hexandiole, Oktandiole oder Mischungen von allen vorgenannten in einer (Gesamt-) Menge von vor allem 5 bis 15 Gew.%. Besonders bevorzugt sind Ethanol, lsopropanol, Propylenglykol, oder Mischungen hiervon, oder n-Hexandiol oder Mischungen von Ethanol und Isopropanol.

Die beschriebenen in situ- Haftgel- bildenden Zubereitungen sind insbesondere geeignet zur topischen kosmetischen oder dermatologisch / medizinischen Anwendung auf befeuchtete Haut, vor allem Schleimhäute des Mund-Rachen-Raumes, die des Analbereichs, die des Urogenitaltrakts, insbesondere die der Scheide, sowie bei Erkrankungen des Auges, wie Bindehaut des Auges, sowohl in der Human- als auch in der Veterinär - Medizin.

Anwendungsgemäß können erfindungsgemäße Zubereitungen eingesetzt werden insbesondere zur Behandlung von Gingivitis, Stomatitis aphtosa et ulcerosa, Rhagaden, Soor, Herpes, Parodontose, Parodontitis, Pharyngitis simplex et sicca, Laryngitis, Glossitis, Infekte nach Zahn-Extraktionen und nach oral-chirurgischen Eingriffen, Prothesen-Druckstellen, Plaques, zur Kariesprophylaxe, Foetor ex ore, (im Mundraum); Rhinitis, Sinusitis, Pollinosis im Nasenraum; Thrombophlebitis, Proctitis, Rhagaden, Fissuren, Pruritus, Brennen, Schmerzen, Blutungen und Ekzeme im Analbereich; Fluor vaginalis, die Colpitis, Cervizitis, atrophische Entzündungen, Pelvipathie, Östrogen-Mangel mit Genital-Atropien, lokale Kontrazeptiva sowie Zubereitungen zur Restitution des Scheiden-Milieus (im Scheidenbereich); Entzündungen, Infekte der Bindehaut oder Wundbehandlung derartiger Schleimhautgewebe wie auch Erkrankungen des Auges oder Neurodermitis.

In allen Fällen kann es zu Gewebe-Defekten (Wunden), kommen, sodass auch diese behandelt werden müssen.

Geeignete Wirkstoffe können vor allem ausgewählt werden aus Kamillenöl, Kamillenblätterextrakt, Rosenholzöl, Sandelholzöl, Rosmarinöl, Palma rosa Öl, Myrrhe, Lavendelöl, Hammamelisblätter Extrakt, Teebaumöl, Bergbohnenkraut, Thymianöl, Vitamin A,B,C,D,E,H, oder Mischungen hiervon.

### Nähere Beschreibung der einzelnen Inhaltsstoffe

### 1. Lipoid- Phase

Diese Öl- bzw. Lipoid-Phase wird gemäß vorliegender Erfindung insbesondere im Wesentlichen aus fetten Ölen gebildet.

Die Öle, auch fetten Öle, oder Lipoide, dieser Phase werden vorteilhaft aus folgenden Substanzgruppen ausgewählt:
- Estern von monovalenten C₃-C₃₀-Alkoholen und linearen C₆-C₁₈Carbonsäuren z.B. Isopropyl-Palmitat, Isopropyl-Myristat, Isopropyl-Stearat, Oleyl-Oleat, . Butyl-Oleat, Isononyl-Isonanoat, Isononyl-Stearat, Iso-octyl-Stearat, n-HexylLaurat, Isopropyl-Oleat;
- Ungesättigten und gesättigten C₁₄-C₂₂Fettsäuren natürlicher und synthetischer Herkunft, wie Ölsäure, Linolsäure, Linolensäure, Palmitinsäure;
- Di- C₁₂-C₂₂- Alkyl-Ether und Triglyceride gesättigter und ungesättigter C₁₄-C₂₀Fettsäuren natürlicher und synthetischer Herkunft, wie Oliven-Öl, Mandel Öl, Raps-Öl, Avocado-Öl, Sonnenblumen-Öl, Soja-Öl, Erdnuss-Öl, Capryl /Caprinsäure-Triglycerid, Nachtkerzensamen-Öl, Borretschsamen-Öl, Johannisbeerkern-Öl, Di-Capryl-Ether, Schwarzkümmel-Öl, Inophyllum, Calophyllum, Maiskeim-Öl, Jojoba-Öl, Aprikosenkern-Öl, Wildrosen-Öl,Weizenkeim-Öl und Mischungen davon.
- C₈-C₂₀ -Fett-Alkohole synthetischer und natürlicher Herkunft wie Lauryl-, Linol-, Linolen-, Arachidon-, Palmitin-Alkohol, Lanolin und acetyliertes Lanolin.

Es sind auch Mischungen aus allen genannten Substanzgruppen möglich. Bevorzugt werden als Ölphase ausgewählt: Ester aus monovalenten C₃-C₃₀-Alkoholen mit C₆-C₁₈- Carbonsäuren, ungesättigten und gesättigten C₁₄-C₂₂-Fettsäuren, Di- C₁₂-C₂₂- Alkyl-Ethern und Triglyceriden gesättigter und ungesättigter C₁₄-C₂₀Fettsäuren, C₈-C₂₀ -Fett-Alkoholen.

Besonders bevorzugt sind Nachtkerzensamenöl, Isopropylmyristat, Glyceryl-Cocoat-Citrat-Lactat, Avocado-Öl, Borretschsamen- Öl, Jojoba- Öl, WeizenkeimÖl, oder Mischungen hiervon.

Im Allgemeinen wird die Öl-(Lipoid)phase gemäß vorliegender Erfindung gebildet aus einem oder mehreren Ölen, Lipoiden oder Mischungen hiervon und bedeutet insofern angegebene Menge an einem oder mehreren Ölen. bzw. einem oder mehreren Lipoiden oder Gemischen hiervon.

### 2. Die Emulsions- Emulgatoren

Der oder die Emulsions- Emulgatoren (einzeln oder Mischungen) sind vor allem ausgewählt aus der Gruppe der Polyethylenglykolderivate, vor allem PEG-monoEster und der PEG-di-Ester gesättigter und ungesättigter C₁₂-C₂₀-Fettsäuren sowie Mischungen hiervon. Erstere sind bevorzugt ausgewählt aus PEG(y)-mono-Estem mit y = 2 bis 50. Letztere sind bevorzugt ausgewählt aus PEG(y)-di-Estem mit y = 4 bis 50 und den gleichen o.g. Fettsäuren. Insbesondere geeignet sind PEG(y)-C₁₂-C₂₀-Fettsäuren mit y = 2-40 und mit den unter 1. genannten Fettsäuren, x₁ + x₂ = 1 -10_{,} vorzugsweise bis 10, (R₁, R₂ ≠H).

Insbesondere sind die Fettsäuren der Ester bevorzugt ausgewählt aus Ölsäure, Linolsäure, Linolensäure, Caprinsäuren und Ricinolsäuren.

Geeignet sind ferner PEG(y)-Glycerol- C₁₄-C₂₀-Fettsäurester mit y = 10 bis 50, x₁ + x₂ = 1-4 (R₁, R₂ ≠H), und mit Ölsäure, Laurinsäure und Stearinsäure als bevorzugte Fettsäuren;

Weiterhin geeignet sind PEG(y) -C₁₄-C₂₂. Sorbitanfettsäure- Ester(m) mit y= 4-50, x₁ + x₂ = 1-6 (R₁, R₂ ≠H), insbesondere PEG(y)-Sorbitan- C₁₄-C₂₀-Fettsäurenester(m) mit y = 4 bis 50, x₁ + x₂ = 1 bis 5 und mit Ölsäure, Laurinsäure, Palmitinsäure und Stearinsäure als bevorzugte Fettsäuren.

Weiter sind als derartige Emulgatoren solche der Formel II geeignet, worin A= (O - B-R₂ₓ₁)- bedeutet Bevorzugt ausgewählt sind als Alkohole Glycerol, Ethylen-Glykol, Pentaerythritol, Poly-Ethylen-Glykol und als R₁,₂ C₁₄-C₂₀-Fettsäuren, insbesondere Fettsäureglyceridöle (gemäß vorliegender Erfindung), z. B. (die lipophilen Komponenten) der natürlichen Öle, z.B. essentielle Öle und / oder Hydroxysubstituierte Öle (auch hydriert) wie Castor-Öl, Mais-Öl, Oliven-Öl, Erdnuss-Öl, Palmkern-Öl, Aprikosenkern-Öl, Mandel-Öl, Oleat, Weizenkeimöl.

Insbesondere geeignet sind (PEG)_{y} -Öl-Emulgatoren der Formel PEG(y)-Öl mit y = 4 bis 50 oder Sucrose-Derivate wie nachfolgend genannt.

Weiterhin sind ausgewählt als Emulsions-Emulgatoren MonolPoly-Glyceryl-Fettsäure-Ester sowie deren Mischungen. Hierzu gehören vor allem Poly-Glyceryl(y)-Fettsäuren(m) mit y = 1 bis 10 und mit x = 1 bis 10 und mit den speziellen o.g. (insbesondere bevorzugten) Fettsäuren.

Unter Alkylethern können PEG- C₁₂-C₂₀-Alkyl-Ether, besonders PEG(n)-Alkyl mit n = 2 bis 50 und mit Alkyl = Oleyl, Lauryl, Cetyl, Stearyl gewählt werden.

### 3. Gel-Emulgatoren

Geeignete Gel- Emulgatoren (einzeln oder Mischungen hiervon) sind ausgewählt aus PEG-Derivaten mit einem hydrophilen Teil aus linearen oder / und verzweigten Molekül-Ketten, insbesondere PEG- Ketten, vor allem mit einem HLB- Wert von mindestens 12 bis 19, insbesondere mindestens 14 bis 19.

Dazu gehören PEG(n)-mono- C₁₄-C₂₀-Fettsäure- Ester mit n = 60 bis 1000, insbesondere als Oleat, Stearat, Laurat, Ricinolat. Geeignet sind auch PEG(400)-di-C₁₄-C₂₀-Fettsäure- Ester wie solche von Oleat, Stearat.

Besonders günstig sind auch Reaktionsprodukte aus natürlichen (essentiellen, und / oder (Hydroxy-)substituierten) und hydrierten natürlichen Ölen mit (ethoxylierten) Poly-Ethylenglykol -Alkoholen wie PEG(n)-Öl mit n = 60 bis 200 und mit Öl = Castor-, hydriertes Castor-, Mais-, Oliven-, Erdnuss-, Palmkern-, Mandel-, Aprikosenkemöl.

Weiterhin geeignet sind PEG(n) -Sorbitan- C₁₄-C₂₀-Fettsäure- Ester mit n = 60, 80 und mit insbesondere Laurat, Palmitat, Stearat, Oleat (als Fettsäureteil). Insbesondere geeignet ist auch PEG(n)Penta-Erythryl-tetra-Stearat mit n= 150 und Pentaerythrol als Alkohol.

Geeignete Alkylether-Gel-Emulgatoren werden ausgewählt aus PEG(n)- C₁₂-C₁₈-' Alkyl-Ethem mit n = 60 bis 400, wobei Alkyl bevorzugt gewählt ist aus Oleyl, Stearyl, Lauryl, Cetyl;

Ferner sind geeignet PEG60-Mandelöl-Glycerid, PEG90-Aprikosenkemöl-Glycerid, PEG60-Nachtkerzenöl-Glycerid, PEG60-Maiskeimöl-Glycerid, PEG60-Passiflora-Glycerid, PEG100-Stearat.

Alle Gel-Emulgatoren können in praktisch beliebigen Mischungen qualitativ und quantitativ angewendet werden.

### 4. Alkohole

Alkohole, sofern vorhanden können ausgewählt werden aus der Gruppe umfassend 1-und 2- wertige C₁-C₈-Alkohole. Bevorzugt sind Ethanol, Propanol, Isopropanol, Butanol, Isobutanol, sowie Hexandiole, Oktandiole und Propylenglykol. Diese sind in Mengen von 0, bzw. 0,1 bis 20 Gew.%, bevorzugt 1 bis 15 , insbesondere 1 bis 12 Gew.%, enthalten.

Mischungen aller genannten Alkohole sind ebenfalls möglich.

Bevorzugt liegen 1 bis 15 Gew. % Ethanol, Isopropanol und Propylenglykol oder Mischungen hiervon vor.

### 5. Zusatzstoffe

Als Zusatzstoffe sind insbesondere Penetrations- und Diffusionsverstärker geeignet. Diese sind vor allem ausgewählt aus: Azone, Pyrrolidon, Ölsäure, Linolsäure, Linolensäure, D-Limonen, Menthol, Propylenglykol, Dimethylsulfoxid, Dimethylformamid, Dimethylacetamid. Der Gehalt der Zubereitungen an diesen Verstärkern wird gewählt zwischen 0,1 und 10,0, bevorzugt zwischen 0,5 und 5,0 Gew.%.

Es hat sich auch gezeigt, dass die Penetration über die Perfusion, d.h. den konvektiven Mechanismus, weiter gesteigert werden kann, wenn die Zubereitungen noch mit hyperämisierenden vasodilatorischen durchblutungsfördemden Wirkstof fen wie Capsaicinoide oder Nicotin-Derivate, versehen werden. Da solche Stoffe in der Regel reizen und irritieren (Jucken, Brennen, Schmerzen erzeugen), ist es angebracht, diese Wirkungen mit lokalanästhesierenden Effektoren, wie Polidocanol oder Lidocain, zu koupieren, letztere z.B. mit einem Gehalt von 0,001 bis 10 Gew.%, bevorzugt 0,03 bis 3 Gew.%.

Anti-Oxidantien (insbesondere zur Stabilisation von Doppelbindungen der Komponenten) können ausgewählt werden aus Tocopherol und dessen Estern wie Acetat, sowie Butyl-Hydroxy-Toluol, diese mit einem Gehalt von 0,005 bis 2 Gew.%, bevorzugt von 0,01 bis 0,5 Gew.%.

Salze und niedermolekulare Säuren wie auch Laugen werden ggf. zur Einstellung des pH- Wertes hinzugefügt. Sie werden ausgewählt werden aus Milchsäure, Salzsäure, Natronlauge, Laktat, Bikarbonat, Borat, diese mit einem Gehalt von 0,01 bis 2 Gew. %, insbesondere 0,1 bis 1 Gew.%.

In besonders geeigneten Ausführungsformen sind 0,1 bis 20 Gew.% Zusatzstoffe enthalten. Diese können bevorzugt ausgewählt werden aus o.g. Substanzen, ferner ggf. auch aus Geschmacksstoffen wie Saccharin-Na" amphoteren Tensiden wie Phosphtid- Derivate wie -Lecithin sowie wasserbindenden Lipoiden wie Adepts lanae zur Stabilisation der Zubereitungen und ggf. Konservierungsstoffe wie Benzyl-Alkoniumchlorid, Chlorhexidin-Acetat, alle in vorbeschriebenen Dosierungen.

### 6. Wirkstoffe

Aus den bekannten Wirkstoffgruppen (Pharmaka, Pflanzen-Inhaltsstoffe, wie Extrakte, und ätherische Öle) sind nachfolgende ausgewählt; (im Folgenden nach Wirkungen geordnet):
**a) anti-bakteriell, anti-infektiös, anti-septisch**
   Harnstoff, Thymol, Chlorhexidin, Hexetidin, Benzydamin-HCl, CetylpyridiniumChlorid, Benzalkoniumchlorid, Dequalium-Chlorid, Polyvidon-Jod, Trichlosan, Milchsäure, Octendin, Salicylsäure, Sanquinarin, Xylitol, Saccharin-Na, Metroindazol, Antibiotika, Pflanzenextrakte wie Kamillen-Extrakt, Salbei-Extrakt, Spitzwegerich-, Echinacea purpurea, Hydrocotyle asiatica, Aloe Vera - Extrakt.
   Fette Öle: Nachtkerzen-Samen; Neem;
   Ätherische Öle aus: Bay, Bergbohnenkraut, Cajeput, Cassis-Zimt, Cistrose, Eukalyptus, Geranien, Gewürznelke, Kamille (blau), Campher, Kiefern, Knoblauch, Basilikum, Latschenkiefer, Weißtanne, Rosenholz, Benzoe-Harz, Lavendel, Lavendelsalbei, Lemongras, Lorbeer, Majoran, Manuka, Myrrhe, Nelken, Niauli, Oregano, Patchouli, Perubalsam, Petit Grain, Pfefferminz, Pfeffer, Piment, Salbei, Speik-Lavendel, Teebaum, Thuja, Thymian, Wacholder, Ysop, Zimt, Zitrone, Galbanum, Bergamotte, Rosmarin, Fichtennadel, Palmarosa, Sandelholz, Terpentin. Harze: Perubalsam, Benzoe, Myrrhe, Galbanum.
   Antibiotika wie vor allem: Fusidinsäure, Mupirocin, Sulfadiazin, Erythromycin, Clindamycin, Tetracyclin, Medocyclin, Tyrothricin, Gentamycin, Neomycin, Bacitracin, Chloramphenicol, Polymyxin B, Kanamycin, Amoxyllin, Azidamfenicol, Ofloxacin, Levofloxacin, Ciprofloxacin, Gramicidin, Oxytetracyclin.
b) adstringierend
   Sympathomimetika wie: Tramazolin, Xylometazolin, Oxymetazolin, Tetryzolin, Naphazolin;
   Gerbstoffe: wie Quercus, Stipites dulcamara-Extrakt, Hamamelis-Extrakt, Rhabarberwurzel, Geranien-Öl, Zitronen-Öl, Myrrhe, Cortex Quercus, Radix Rhei, Radix Ratanhiae, Radix Tormentillae, Rhizoma Hydrastis;
   Ätherische Öle und Harze z. B. aus: Geranie, Grapefruit, Mastix, Zitrone, Myrrhe, Öl- Extrakt von Johanniskraut.
c)analgetisch
   Carbonsäuren wie Salicylsäure, Diflunisal, Salicylamid, Ethenz-Amid, Acetyl-Salicylsäure, Salsalat, Benzydiamin-HCl, Methyl-Salicylat, Hydroxy-ethyl-Salicylat; Essigsäure-Derivate wie : Iridometacin, Acemetacin, Proglumetacin, Diclofenac, Tolmetin, Lonazolac, Fenbufen, Nabumeton.
   Propionsäure-Derivate wie Iboprofen, Ketoprofen, Flurbiprofen, Tiaprofensäure, Fenoprofen, Naproxen, Dexketoprofen.
   Heterozyklische Keto-Enol-Säuren wie Oxicame ausgewählt, insbesondere: Piroxicam, Tenoxicam, Metoxicam, Meloxicam, Lomexicam.
   Anthranilsäure-Derivate wie: Mefenaminsäure, Flufenaminsäure, Nifluminsäure. Nabumeton, Azapropazon, Aceclofenac.
   Pyrazolidone wie: Oxyphenbutazon, Phenylbutazon, Mofebutazon.
   Pyrazolinone wie: Propylphenazon, Metamizol; Ferner Paracetamol, Bufexamac, Capsaicinoide (Capsaicin, Dihydrocapsaicin, Nordihydro-Capsaicin, Homodihydro-Capsaicin, Homo-Capsaicin, Nonivamid); Extrakte aus Beinwell, Weidenrinden, Stiefmütterchenkraut, Spierblumenkraut; Ätherische Öle aus: Johanniskraut, Basilikum, Bergbohnenkraut, Birke, Fenchel, Fichtennadel, Ingwer, Kamille, Campher, Lavendel, Lavendelsalbei, Lorbeer, Majoran, Melisse, Niaouli, Oregano, Patchouli, Pfeffer, Pfefferminz, Rainfarn, Rosmarin, Sade-Baum, Schafgarbe, Speik-Lavendel, Teebaum, Thymian, Wintergrün.
d) anästhesierend
   Ester-Lokalanästhetika wie Benzocain, Procain, Tetracain, Thymol, Polidocanol. Amid-Lokalanästhetika wie speziell Prilocain, Mepivacain, Lidocain, Etidocain, Bupivacain, Levobupivacain, Ropivacain, Articain, Fomocain.
   Ätherische Öle wie: Campher, Pfefferminz, Thymian.
e) antihämorrhagisch, phlebotonisch
   Sparteinsulfat, Dihydro-Ergotamin, Cumarin;
   Extrakte aus: Hamamelis, Ruscus aculeatus, Melilotus albus, Rotes Weinlaub, Aesculus hippocastanum, Melilotus officinalis, Cortex Quercus, Tormentilla Rhizoma, Mahonia, Calendula, Cardiospermum, Centella;
   Ätherische Öle aus : Texas-Zeder, Virginea-Zeder, Cistrose, Basilikum, Cajeput, Geranie, Melisse, Neroli, Niaouli, Rainfam, Teebaum, Zitrone, Zypresse, Weihrauch, Patchouli, Sandelholz, Lavendel, Myrte, Schafgarbe, Immortelle, Cistrose, Geranie, Mastix, Melisse, Muskatellersalbei.
f) anti-histaminisch, anti-allergisch
   Schwarzkümmel-Öl, Rainfarn, Cromoglycin-Säure, Lodoxamid. Glyccyrrhitinsäure, Azelastin-HCl, Naphazolin-HCl, Xylometazolin-HCl, Oxymetazolin-HCl, Tetryzolin, Zink-lonen (Zinksulfat);
   Glukokorticoide: Budenosid, Betaclomethason-di-Propionat, Dexamethason, Fluticason, Momethason, Triamcinolon, Betamethason, Prednisolon.
g) anti-koagulierend
   Anti-Styptika sind Hirudin, Hirudin-Derivate, Heparine, insbesondere niedermolekulare Öle wie: Immortelle, Zimt, Lavendel, Lorbeer, Zitrone.
h) anti-mykotisch
   Azol-Derivate wie vor allem: Clotrimazol, Bifonazol, Econazol, Fenticonazol, Isoconazol, Oxiconazol, Sertaconazol, Tioconazol, Miconazol, Ketoconazol, Itraconazol, Fluconazol, Voriconazol.
   Squalen-Epoxidase-Hemmer wie Terbinafin und Naftfin.
   Morpholine wie Amorolfin;
   Weiterhin auch Amphotericin B, Griseofulvin, Flucytosin, Ciclopirox, Nystatin, Natamycin sowie Thiocarbonate; oder
   Stipites dulcamara -Extrakt; oder Öle wie: Lavendel, Lavandula spicca, Manuka, Nelke, Niaouli, Oregano, Gingergras, Patchouli, Pfefferminz, Bay, Piment, Bergbohnenkraut, Cassia-Zimt, Zimt, Eukalyptus, Geranie, Gewürznelke, Ho-Baum,Campher, Piment, Rosmarin, Salbei, Speik-Lavendel, Tagetes, Teebaum, Thymian, Ravensara, Rosenholz- Öl, Palma rosa.
i) anti-ödematös
   Aesculus hippocastanum, Ruscus aculeatus, Melilotus officinalis, Fagopyrum esculentum, Roter Weinlaub, Solidago virgaurea.
   Ätherische Öle wie: Baldrian, Basilikum, Fenchel, Geranie, Campher, Kiefer, Lorbeer, Majoran, Patchouli, Pfeffer, Rosmarin, Sandelholz, Schwarzkümmel, Texas-Zeder, Thuja, Wacholder, Zypresse.
j) anti-parasitisch
   Ausgewählt aus Pharmaka ist besonders Crotamiton.
   Ausgewählt aus den ätherischen Ölen sind Pfefferminz, Zimt, Teebaum-Öl.
k) anti-phlogistisch
   Steroidale Antiphlogistika wie vor allem Glukokortikoide; Betamethason, Dexamethason, Prednisolon, Hydrocortison; oder auch Bufexamac, Glycyrrhetin-Säure. Thymol, Cavacrol, Campher, Eugenol, Zimt-Aldehyd, Dexpanthenol; oder
   Anti-Phlogistika aus Pflanzen-Extrakten wie: Melilotus officinalis, Rusculus aculeatus, Aesculus hippocastanum, Johanniskraut, Ringelblume, Aloe vera, Nachtkerzensamen, Borretschsamen, Symphytum, Amika;
   Gerbstoffe wie speziell Quercus-Extrakt und entsprechende Synthetika wie Tannine sowie Catechine, Stipites dulcamara, Hamamelis, Radix Rhei, Radix Ratantiae, Radix Tormentillae;
   Öle wie Johanniskraut, Kamille blau, Kamille römisch, Latschenkiefer, Lavendel, Lavendel extra, Lemongras, Basilikum, Benzoeharz, Birke, Campher, Eukalyptus, Fichtenadel, Galbanum, Schwarzkümmel, Teebaum, Thymian, Weihrauch, Wintergrün, Ysop, Zimt, Manuka, Nelke, Palma rosa, Patchouli, Petit Grain, PeruBalsam, Rosmarin, Schafgarbe, Rainfam, Myrrhe, Citronella-Gras, Kalmus, Koriander, Limette, Verbene.
I) anti-spastisch, relaxierend
   Ätherische Öle z. B. aus: Basilikum, Birke, Lavendelsalbei, Lorbeer, Majoran, Petit Grain, Wintergrün, Pfefferminz, Campher, Fichtennadel, Galgant, Stemanis, Styrax, Citronella, Muskatellersalbei, römische Kamille, Limette, Petit Grain, Ylang Ylang, Weinraute.
   Harze: Perubalsam, Galbanum.
m) anti-viral
   Ausgewählt aus der Gruppe der Pharmaka sind: Trifluridin, Vidarabin, Tromantadin, Foscamet, beta-Interferon, Aciclovir, Valciclovir, Penciclovir, Famciclovir, Idoxariden, Bivudin.
   Ausgewählt aus Extrakten sind: Podophyllum (Podophyllin), Melissenblätter, Fructus capsici, Capsaicinoide (Capsaicin, Dihydrocapsaicin, Nordihydro-Capsaicin, Homodihydro-Capsaicin, Homo-Capsaicin, Nonivamid).
   Ausgewählt aus ätherischen Ölen sind: Ysop, Ravensara aromatica, Piment, Pfefferminz, Pfeffer, Oreganon, Niaouli, Cistrose, Cassia-Zimt, Cajeput, Bay, Basilikum, Melisse, Zitronengras, Zimt, Manuka, Campher, Lavendel, Ho-Baum, Gewürzkümmel, Eukatyptus, Zitrone, Thymian, Thuja, Teebaum, Speik-Lavendel, Salbei;
   Harze: Myrrhe.
n) regenerierend, wundheilend, pflegend
   Hier eignen sich Hormone und Vitamine wie: Vit. E, C, F, D, B, H und A, Estradiol Progesteron und Estriol sowie Mischungen hiervon;
   Dexpanthenol, aplpha-Bisabolol, Bibrocathol;
   Extrakte (Ölige bzw. wässrige bzw. alkoholische bzw. mechanisch gewonnene) aus Wildrose, Mandel, Weizenkeim, Avocado, Aloe vera, Borretschsamen, Jojoba, Sheabutter, Nachtkerzensamen, Olive, Amika, Calendula, Kamille, Symphetum, Hyperici, Gerbstoffe wie Hamamelis; Salze: insbesondere für die Zähne: Aminfluorid, Zinnfluorid, Natrium-, Kaliumfluorid, Kaliumnitrit, Phosphat;
   Ätherische Öle z. B. aus Kamille, Angelika, Baldrian, Bergamotte, Geranie, Manuka, Vetiver, Thuja, Johanniskraut.
   Harze: Myrrhe, Benzoe, Weihrauch, Galbanum.
o) hyperamisierend
   Aus der Gruppe der Pharmaka sind besonders ausgewählt: Nicotinsalicylsäure-Ester, Capsaicinoide (Capsaicin, Dihydrocapsaicin, Nordihydro-Capsaicin, Homodihydro-Capsaicin, Homo-Capsaicin, Nonivamid), Benzyl-Nicotinat.
   Aus den Pflanzen-Extrakten sind ausgewählt: Amika, Erdnuss, Oliven.
   Aus den ätherischen Ölen sind ausgewählt: Cassia-Zimt, Birke, Ingwer, Campher, Kiefern, Oregano, schwarzer Pfeffer, Rosmarin, Sade-Baum, Vetiver, Weißtanne, Zimt.
p) immun-modulatorisch
   Extrakte aus Echinacea, Calendula, Chamomilla recutita, Viscum album, Eleuthe-Rococcus Eupatikum, Stipites dulcamara, Viola tricolour; Similax americana, Glycyrrhiza glabra.
   Ätherische Öle aus: Vetiver, Nelke, Salbei, Thymian, Teebaum, Patchouli, Oregano, Campher, Zimt, Zitronengras, Melisse, Niaouli.
   Harze: Myrrhe, Weihrauch.
g) juckreizstillend
   Aus der Gruppe der Pharmaka sind besonders ausgewählt: Bufexamac, synthetische Gerbstoffe, Polidocanol, Lidocain, Glycyrrhitinsäure.
   Von den Extrakten sind ausgewählt Rosskastanie, Kamillenblüten, Melilotus officinalis, Ruscus aculeatus, Fructus capsici, Capsicum (Capsaicin), Borretschsamen, Avocado, Nachtkerzensamen, Wildrose, Gerbstoffe aus Quercus, Hamamelis. Von den ätherischen Ölen sind speziell ausgewählt: Pfefferminz, Lavendel, Manuka, Rainfam, Bergamotte.
r) vasodilatorisch
   Von den Pharmaka sind Nitroglycerin und Benzyl-Nicotinat besonders ausgewählt. Von den Extrakten ist besonders Weißdorn ausgewählt.
   Von den ätherischen Ölen ist speziell Birke, Lemongras und Wintergrün ausgewählt.
s) anti-oxidativ
   Aus den Pharmaka sind ausgewählt: Selen, Mangan, Kupfer, 1-Glutathion, I-Cystein, Co-Enzym Q 10, alpha-Liponsäure, Butyl-Hydroxy-Toluol.
   Von den Naturstoffen sind ausgewählt: Vitamin A, C und E, Zeaxanthin, Lycopin, beta-Carotin, Carotinoide, Pro-Anthoxy-Cyanidine, Anthocyane, Flaconoide.
t) befeuchtend
   Von den Wasserbindem sind ausgewählt: Hyaluronsäure, Hamstoff, Pyrrolidoncarbonsäure, Lactat, Aloe vera, Glycerol, Hexylenglykol.
u) Beta-Adrenorezeptoren-Blocker:
   Betaxolol, Carteolol, Levobunolol, Metipranolol, Timolol.
v) Sympathomimetika
   Apraclonidin, Brimonidin, Clonidin, Dipivefrin.
w) Parasymnathomimetika:
   Carbachol, Pilocarpin.
x) Carboanhydrase-Hemmer
   Acetazolamid, Brinzolamid, Dorzolamid.
y) Prostaglanin-Derivate
   Latanoprost
z) Kontrazeptiva
   Nonoximol

Die Wirkstoffe können in allgemein bekannten Mengen /Dosierungen in die Zubereitungen eingearbeitet werden. Der Gehalt der verschiedenen Wirkstoffe in den Zubereitungen gemäß den nachfolgenden Beispielen wurde dementsprechend an die bekannten Einmal-Dosierungen angepasst, unter der Annahme, dass jeweils etwa 2 ml der entsprechenden Zubereitung zur Anwendung kommen, speziell in der Scheide 5 ml, für das Auge nur einige Tropfen.

### Herstellung der erfindungsgemäßen Zubereitungen

Die erfindungsgemäßen Zubereitungen werden hergestellt, indem der Lipoid(Öl)phase der Mikro-Emulsion, welcher der /die Emulsionsemulgator(en) zugesetzt werden (und somit den oder die Emulsions- Emulgatoren dann bereits aufweist), der Gel-Emulgator zugesetzt wird. Zu dieser Mischung wird Wasser oder wässrige Lösungen und ggf. Alkohol, z.B. im Gemisch mit Wasser / wässrigen Lösungen, in den jeweils angegebenen Mengen, hinzugesetzt. In diese Gesamt-Mischung werden schließlich die vorgesehenen Wirkstoffe / Zusatzstoffe für die betreffende Anwendung (Indikation) eingearbeitet.

### Indikationen und Behandlungen

Die erfindungsgemäßen Zubereitungen eignen sich insbesondere für die Anwendung als bzw. Herstellung von kosmetischen und dermatologischen Mitteln zur topischen Behandlung von Haut, befeuchteter Haut, Schleimhäuten. Befeuchtete Haut kann dabei solche sein, die durch Schweißbildung oder Sekretbildung entsteht. Als Schleimhäute kommen die des Mund-Rachen-Raumes, die des Analbereichs, die des Urogenitaltrakts, insbesondere die der Scheide, sowie die Bindehaut des Auges in Betracht. Das Auftragen kann manuell durch Einreiben oder Sprühen erfolgen. Die Zubereitungen sind insbesondere pH-Wert- Hautkompatibel, d.h. auf einen Wert von insbesondere zwischen 6,5 bis 6,9 eingestellt.

Im Mund-Rachen-Bereich steht an erster Stelle die Karies-Prophylaxe. Insbesondere sind zu behandeln: Stomatitis aphtosa et candida, Herpes labialis, Parodontopathien wie Gingivitis, Parodontitis apicalis, Stromatitis ulcerosa et angularis, Prothesen- Druckstellen, Foetor ex ore, Rhagaden, Pharyngitis simplex et sicca, Laryngitis, Glossitis, Infekte nach Zahn-Extraktionen und nach oral-chirurgischen Eingriffen, Plaques, aber auch das Auge selbst.

Im Nasenraum sind vorwiegend infektiöse (bakterielle und virale) und allergische Entzündungen (Rhinitis, Sinusitis, Pollinosis) zu bekämpfen.

Im Analbereich sind meist Entzündungen (Thrombophlebitis, Proctitis, Rhagaden, Fissuren), Pruritus, Brennen, Schmerzen, Blutungen und Ekzeme zu behandeln. Für die Behandlung der Scheide kommen insbesondere hormonale, anti-bakterielle, anti-virale, anti-mykotische und anti-phlogistische Therapien (Vaginitiden) in Frage, speziell der Fluor vaginalis, die Colpitis, Cervizitis, atrophische Entzündungen, Pelvipathie, Östrogen-Mangel mit Genital-Atropien, lokale Kontrazeptiva sowie Zubereitungen zur Restitution des Scheiden-Milieus.

Für die Behandlung von Augenerkrankungen, insbesondere der Bindehaut, kommen vor allem jegliche Art von Entzündungen in Betracht, aber auch Infekte.

In allen Fällen kann es zu Gewebe-Defekten, sprich Wunden, kommen, sodass auch diese behandelt werden müssen.

Das aktive Haftgel kann auch mit Vorteil auf äußerer Haut angewendet werden, z. B. wenn diese sich selbst anfeuchtet. Das passiert, insbesondere bei lokalen Entzündungen, die ganz unterschiedlicher Genese sein können, wie Neurodermitis, Akne, Rosacea, Psoriasis, Mykose, Ekzem, Haut-Degenerationen, insbesondere bei Diabetes mellitus. Der Vorteil besteht darin, dass das aktive Haftgel die entsprechenden Wirkstoffe lange Zeit lokal vorhalten kann.

Generell sind die Zubereitungen aufgrund ihrer allgemeinen Zusammensetzung anti-bakteriell, anti-viral, anti-mykotisch und indirekt auch anti-phlogistisch wirksam. Mit Hilfe eingearbeiteter Effektoren (Wirkstoffe) ist darüber hinaus das gesamte pharmazeutisch-therapeutische Spektrum realisierbar, sowohl in kosmetischer als auch in medizinischer Hinsicht. Speziell sind hier u.a. zu nennen adstringierende, ant-allergische, wundheilende, anti-oxidative, anästhesierende, antiparasitische, analgetische, hormonale, spermicide, hyperämisierende und desinfizierende Wirkungen.

Die Zubereitungen können sowohl therapeutisch und präventiv als auch kausal und symptomatisch eingesetzt werden, und eigenen sich für die kosmetische und die arzneiliche Anwendung.

Die Menge des verabreichten Mittels und der Dosierungsplan zur Prävention oder Behandlung eines wie oben beschriebenen (medizinischen) Zustandes mit der erfindungsgemäßen Zubereitung hängen bei einer medizinischen Indikation von einer Vielzahl von Faktoren ab, einschließlich dem Alter, Gewicht, Geschlecht und Zustand des Patienten, der Schwere der Störung, der Verabreichungsroute und der jeweiligen verwendeten Verbindung, und kann deshalb weitestgehend schwanken. Die erforderliche oder gewünschte Dosis kann auf ein- oder mehrere Male täglich, insbesondere 1 bis 3 mal verteilt werden. Bei einer nicht medizinischen oder dermatologischen / kosmetischen Anwendung werden dem Fachmann bekannte Mengen verabreicht, so oft, und solange nötig oder gewünscht. Insbesondere können erfindungsgemäße Zubereitungen angewendet werden (z.B. als dermatologische / Arzneimittel), vor allem zur Behandlung folgender Indikationen:
i) Karies -Prophylaxe
   Dazu werden Wirkstoffe aus den Gruppen 6a, 6h, 6k, 6m, 6n, 6p, und 6s verwendet.
   Bevorzugt werden eingesetzt: alpha-Bisabolol, Dexpanthenol. Allantoin, Vitamin C und E, BHT, Extrakte: Chamomillae, Calendula, Äther. Öle: Lavendel, Cassia-Zimt, Gewürznelke, Manuka, Bergbohne, Oregano, Salbei, Thymian, Pfefferminz; Myrrhe, Saccharin-Na, Chlorhexidin, Xylitol, Aminfluorid, Cetyl-Pyridinum-Chlorid, Natriumfluorid, Phosphat.
ii) Stomatitis Candida (Mundsoor)
   Dazu werden Wirkstoff aus den Gruppen 6a, 6h, 6k, 6n, 6s eingesetzt.
   Bevorzugt werden eingesetzt:
   Chlorhexidin, Tyrothricin, Amphotericin B, Nystatin, Natamycin, Miconazol, Ketoconazol; Äther. Öle: Manuka, Gewürznelke, Salbei, Cassia-Zimt, Bergbohne, Oregano, Niaouli, Bay, Pfefferminz; Myrrhe, Extrakte : Kamille, Rhei; weiterhin Dexpanthenol, Allantoin, Vit. E, C.
iii) Stomatitis aphtosa, Herpes labialis
   Dazu werden Wirkstoffe aus den Gruppen 6a, 6b, 6d, 6h, 6k, 6m, 6n, 6s verwendet.
   Bevorzugt werden:
   Chlorhexidin, Cetyl-Pyridinum-Chlorid (CPC), Aciclovir, Polidocanol, Lidocain Äther. Öle: Cassia-Zimt, Melisse, Bay, Teebaum, Niaouli, Salbei, Campher.
   Harze: Myrrhe, Benzoe; Extrakte.: Hydrastis canadensis, Rhei, Radix Ratanhiae Glycyrrhitin-Säure, Folia Melissae, Dexpanthenol, alpha-Bisabolol, Allantoin, Vit. A, E, BHT.
iv) Parodonttopathien: Gingivits, Parodontitis apicalis, Stomatitis uloerosa, Stomatitis angularis, Foetotr ex ore; Prothesen - Druckstellen, Wunden nach chirurgischen Eingriffen:
   Dazu werden Wirkstoffe aus den Gruppen 6a, 6b, 6c, 6d, 6f, 6h, 6j, 6k, 6m, 6n, 6s verwendet. Bevorzugt sind:
   Chlorhexidin, Benzalkonium-Chlorid, Salicylsäure, Triclosan, Clindamycin, Tyrothricin, Aciclovir, Metronidazol, Pölidocanol, Lidocain, Benzocain, Campher, Flurbiprofen, Prednisolon- Acetat, Gycyrrhitinsäure, Allantoin, Dexpanthenol, alpha-Bisabolol;
   Äther. Öle: Gewürznelke, Cassia-Zimt, Thymian, Oregano, Palmarosa, Pfefferminz, Wintergrün, Salbei, Bay, Manuka, Lorbeer, Niaouli.
   Harze: Benzoe, Myrrhe, Weihrauch; Extr.: Kamille, Calendula, Amika, Radix Ratanhiae, Rhei, Rhiz. Hydrastis, Radix Tormentillae, Vit E, BHT.
v) Pharvngftis, Laryngitis, Rhinits, Sinusitis
   Dazu werden Wirkstoffe aus den Gruppen 6a, 6d, 6f, 6h, 6k, 6m, 6n, 6t verwendet. Bevorzugt sind:
   Amoxicilin, Chlorhexidin, CPC, Glycyrrhitin-Säure, Budenosid, Beclometason, Dexamethason, Fluticason, Mometason, Triamcinolon, Na-Cromoglicat, Azelastin-HCl, Naphazolin-HCl, Oxymetacolin, Xylometacolin, Benzocain, Dexoanthenol, alpha-Bisabolol, Aloe vera, Vit. E, Lycopin.
   Äther. Öle: Campher, Niaouli, Bay, Eucalyptus, Latschenkiefer, Bergkiefer, Oregano, Gewürznelke, Majoran, Palmarosa, Pfefferminz, Salbei, Thymian, Gingergras, Ravensara; Harze: Benzoe, Myrte, Myrrhe.
vi) Hämorrhoiden
   Dazu werden Wirkstoffe aus den Gruppen 6b, 6c, 6d, 6e, 6f, 6g, 6i, 6k, 6l, 6q, 6s verwendet. Bevorzugt sind:
   Benzocain, Cinchocain, Lidocain, Policresulen, Polidocanol, Prednisolon, Glycyrrhi-tinsäure, Bufexamac, Cumarin, Hirudin, Aloe vera, Perubalsam, Mastix, Galbanum.
   Extr.: Hamamelis, Rosskastanie, Steinklee, Hydrocotyle asiatica, Johanniskraut-Öl Äther. Öle: Wacholder, Zypresse, Rosmarin, Campher, Sandelholz, Cajeput, Geranie, Patchouli, Niaouli, Myrte.
vii) Vaginitiden: Flour vaginalis, Colpititis, Pelvipathie; Scheidenkatarrh
   Dazu werden Wirkstoffe aus den Gruppen 6a, 6b, 6d, 6h, 6k, 61, 6m, 6n, 6o, 6q, 6r, 6s, 6t verwendet. Bevorzugt sind:
   Clindamycin. Neomycin, Tetracyclin, Clotrimazol, Miconazol-Nitrat, Fluconazol, Nystatin, Amphtericin B, Fenticonazol, Milchsäure, Hexetidin, Policresulen, Octenidin, Dequalinium-Chlorid, Metronidazol, Aciclovir, Polidocanol, Benzyl-Nicotinat Extr.: Quercus, Kamille, Salbei, Aloe vera.
   Äther. Öle.: Palmarosa, Cassia-Zimt, Oregano, Lavendel, Thymian, Bergbohne, Gingergras, Rosmarin, Teebaum, Niaouli, Melisse, Ravensara, Rosenholz; Dexpanthenol, alpha-Bisabolol, Ichthyiol, Perubalsam, Vit. E, BHT, Estriol, Estradiol, Progesteron.
viii) Ophthalmika: Konjunktivismus, Blepharitis
   Dazu werden Wirkstoffe aus den Gruppen 6a, 6b, 6d, 6f, 6h, 6k, 6m, 6n, 6t verwendet. Bevorzugt sind:
   Fusidinsäure, Gentamycin, Kanamycin, Bacitracin, Gramicidin, Naphazolin, Tetryzolin, Tetracain, Oxybuprocain, Azelastin-HCl, Na-Cromoglicat, Betamethason, Prednisolon , Dexamethason, Natamycin, Polymyxin B, Dexpanthenol, Vit. A und E, Zn-Sulfat, Aciclovir, Bibrocathol, Povidon, Lavendel, Kamille blau.
ix) Glaukom
   Dazu werden Wirkstoffe aus den Gruppen 6u, 6v, 6w, 6x, 6y verwendet. Bevorzugt sind: Timolol, Clonidin, Pilocarpin, Dorzolamid, Latanoprost.
x) Neurodermitis
   Dazu werden Wirkstoffe aus den Gruppen 6a, 6d, 6e, 6f, 6k, 6n, 6p, 6q, 6s, 6t verwendet.
   Bevorzugt sind: Urea pura, Glycyrrhitinsäure, Cardiospermum, Nachtkerzensamen-Öl, Kamille, Hamamelis, Geranien-Öl, Lavendel-Öl, Rosenholz-Öl, Sandelholz-Öl.

### Beispiele

Die Erfindung wird anhand nachfolgender Beispiele 1-34 näher erläutert. Beispiele 1-22 betreffen erfindungsgemäße Zubereitungen (Basis-Gele), Beispiele 23-34 Anwendungszubereitungen und Anwendungen hiervon.

Folgende Zubereitungen 1-22 wurden wie beschrieben hergestellt und sind in Tabelle 1a, b, 2 a, b wiedergegeben (Die % Angaben bedeuten Massen(M)-Prozente).

**Tabelle 1a (Beispiele 1-6)**

| Gel-Basis | 1 | 2 | 3 | 4 | 5 | 6 |
|---|---|---|---|---|---|---|
| Isopropyl-Myristat | 44,99 | 20,00 | | | | |
| Polysorbat 80 | 29,42 | 37,50 | 37,60 | 38,60 | 41,00 | 37,00 |
| Ethanol 96% | 6,48 | 4,00 | 4,00 | 4,00 | 4,00 | 4,00 |
| Limonen | 2,00 | | | | | |
| Aqua dest. | 9,54 | 4,00 | 4,00 | 4,00 | 4,00 | 4,00 |
| α-Tocopherol-Acetat | 0,03 | 0,03 | 0,03 | 0,03 | 0,03 | 0,03 |
| acetyl, Lanolin-Alkohol PA | | 18,60 | 39,00 | | | |
| PEG60-Mandel-Glyceride | | 6,00 | 6,00 | 5,00 | 6,00 | 6,00 |
| PEG35 Castor-Öl | | 6,40 | 6,00 | 5,00 | 6,00 | 3,00 |
| Nachtkerzen-samen-Öl | | 3,00 | 3,00 | 3,00 | | 3,00 |
| acetyl. Lanolin-Alkohol CR | | | | 38,00 | 38,40 | 39,40 |
| PEG6-Caprat/ Caprylat-Glycer. | | | | 2,00 | | 3,00 |
| PEG150Erythityl4 Stearat4 | | | | 0,37 | 0,20 | 0,30 |
| Lecithin | | | | | | |
| PEG40-hydr. Castor-Öl | | | | | | |
| Butyl-Hydroxy-Toluol | | | | | | |
| Glyceryl-Cocoat-Citrat-Lactat | | | | | | |
| Glyceryl-Caprylat | 7,22 | | | | | |
| DMSO | | | | | | |
| Polidocanol | | | | | | |
| Adeps lanae | | | | | | |
| Propylenglykol | | | | | | |
| Polyglyceryl3-poly-Ricinoleat | | | | | | |
| PEG90-Aprikosenkem-Öl-Glycerid | 0,50 | | 0,30 | | 0,37 | |
| PEG100-Stearat | | 0,47 | 0,27 | | | 0,27 |
| Peg60-Passiflora-Glycerid | | | | | | |

**Tabelle 1 b) Beispiele 7-11**

| Gel-Basis | 7 | 8 | 9 | 10 | 11 |
|---|---|---|---|---|---|
| Isopropyl-Myristat | | 19,60 | 17,00 | 19,34 | 20.74 |
| Polysorbat 80 | 33,80 | 44,00 | 42,00 | 44,00 | 44,00 |
| Ethanol 96% | 4,00 | 5,40 | 5,40 | 5,37 | 5,37 |
| Limonen | | 2, 00 | 2,00 | 2,00 | 2,00 |
| Aqua dest. | 4,00 | 5,20 | 5,20 | 5,20 | 6,00 |
| α-Tocopherol-Acetat | 0,03 | 0,03 | 0,03 | 0,03 | 0,03 |
| acetyl, Lanolin-Alkohol PA | 37,00 | | | | |
| PEG60-Mandel-Glyceride | 6,00 | | | | |
| PEG35 Castor-Öl | 6,00 | | | | |
| Nachtkerzensamen-Öl | 3,00 | | 3,00 | | |
| acetyl. Lanolin-Alkohol CR | | 20,00 | 20,00 | 20,00 | 20,00 |
| PEG6-Caprat/ Caprylat-Glycer. | 3,00 | 1,80 | 3,60 | | |
| PEG150Erythrityl4 Stearat4 | 0,20 | 0,20 | 0,40 | | 0,45 |
| Lecithin | | 1,40 | 1,40 | 1,40 | 1,40 |
| PEG40-hydr. Castor-Öl | | | | 2,00 | |
| Butyl-Hydroxy-Toluol | | | | 0,01 | 0,01 |
| Glyceryl-Cocoat-Citrat-Lactat | | | | | |
| Glyceryl-Caprylat | | | | | |
| DMSO | | | | | |
| Polidocanol | | | | | |
| Adepslanae | | | | | |
| Propylenglykol | | | | | |
| Polyglyceryl3-poly-Ricinoleat | | | | | |
| PEG90-Aprikosenkem-Öl-Glycerid | | | | | |
| PEG100-Stearat | | 0,37 | 0,65 | 0,65 | |
| Peg60-Passifloraglycerid | 2,97 | | | | |

**Anwendungsbeispiele 12-17 (Tabelle 2a)**

| Beispiel Gel | 12 | 13 | 14 | 15 | 16 | 17 |
|---|---|---|---|---|---|---|
| Isopropyl-Myristat | | 45,67 | 43,87 | 43,52 | 35,70 | 41,45 |
| Polysorbat 80 | 29,67 | 28,90 | 27,40 | 27,40 | 27,40 | 26,00 |
| Ethanol 96% | 9,00 | 6,22 | 6,00 | 6,00 | 6,00 | 5,50 |
| Limonen | | 2,00 | 2,00 | 2,00 | 2,00 | 2,00 |
| Aqua dest. | 9,00 | 8,36 | 7,60 | 7,60 | 7,60 | 7,00 |
| α-Tocopherol-Acetat | 0,03 | 0.03 | 0,03 | 0,03 | 0,03 | 0,03 |
| acetyl, Lanolin-Alkohol PA | | | | | | |
| PEG60-Mandel-Glyceride | | | | | | |
| PEG35 Castor-Öl | | | | | | |
| Nachtkerzensamen-Öl | | | | | | |
| acetyl. Lanolin-Alkohol CR | 44,00 ' | | | | | |
| PEG6-Caprat/ Caprylat-Glycer. | 1,80 | | | | | |
| PEG150Erythrityl4 Stearat4 | 0,20 | 0,10 | | 0,45 | | |
| Lecithin | | | | | | |
| PEG40-hydr. Castor-Öl | | | | | | |
| Butyl-Hydroxy-Toluol | | | | | | |
| Glyceryl-Cocoat-Citrat-Lactat | 3,00 | 7,22 | | | 7,00 | |
| Glyceryl-Caprylat | 3,00 | | 7,00 | 7,00 | | 6,50 |
| DMSO | | | 5,00 | 5,00 | 5,00 | |
| Polidocanol | | | | 1,00 | 1,00 | |
| Adeps lanae | | | | | 3,00 | |
| Propylenglykol | | | | | | 11,00 |
| Polyglyceryl3-poly-Ricinoleat | | | | | | |
| PEG90-Aprikosenkem-Öl-Glycerid | 0,3 | | 1,10 | | 0,30 | 0,27 |
| PEG100-Stearat | | 1,50 | | | 0,25 | 0,25 |
| Peg60-Passiflora-Glycerid | | | | | 4,97 | |

**Anwendungsbeispiele 18-22 (Tabelle 2b)**

| Beispiel Gel | 18 | 19 | 20 | 21 | 22 |
|---|---|---|---|---|---|
| Isopropyl-Myristat | | | | 46,50 | 42,40 |
| Polysorbat 80 | 34,00 | 34,30 | 29,50 | 34,00 | 34,00 |
| Ethanol 96% | 10,00 | 14,00 | 5.00 | 0,60 | 0,60 |
| Limonen | | | | | |
| Aqua dest. | 6,00 | 10,00 | 10,00 | 4,20 | 4,20 |
| α-Tocopherol-Acetat | 0,03 | 0,03 | 0,03 | 0,03 | 0,03 |
| acetyl, Lanolin-Alkohol PA | | | | | |
| PEG60-Mandel-Glyceride | | | | | 3,00 |
| PEG35 Castor-Öl | | | | | |
| Nachtkerzensamen-Öl | | | | | |
| acetyl. Lanolin-Alkohol CR | 43,50 | 38,00 | 42,00 | | |
| PEG6-Caprat/ Caprylat-Glycer. | | | 13,00 | | 3,00 |
| PEG150Erythrityl4 Stearat4 | 0,47 | | 0,52 | 0,12 | |
| Lecithin | | | | 1,40 | 1,40 |
| PEG40-hydr. Castor-Öl | | | | | |
| Butyl-Hydroxy-Toluol | | | | | |
| Glyceryl-Coooat-Citrat-Lactat | 3,00 | | | | |
| Glyceryl-Caprylat | 3,00 | | | | |
| DMSO | | | | 8,00 | 6,00 |
| Polidocanol | | | | | |
| Adeps lanae | | | | | |
| Polygtyocryl3-poly-Ricinoleat | | | | 4,80 | 4,80 |
| PEG90-Aprikosenkern-Öl-Glycerid | | | | | 0,57 |
| PEG100-Stearat | | 0,17 | | 0,35 | 0,20 |
| Peg60-Passiflora-Glycerid | | 3,50 | | | |

### Anwendungsbeispiel 23

In das Basis-Gel 4 (vergl. Tabelle 1a) wurden eingearbeitet (in M%): 2,0 Extr. Chamomillae Fluid 1:1; 2,0 Calendulae Fluid 1:1; jeweils 0,36 Ol. Lavandulae; Ol. Caryophylli; Ol. Thymii: Ol. Origanae; Ol. Saturejae; Ol. Cassiae; 0,56 Ol. Menthae piperitae; 0,24 Ol. Salviae; 0,24 Ol. Manukae; 0,5 Myrrhe; 0,05 Saccharin.

### Anwendungsbeispiel 24

In das Basis-Gel 9 (vergl. Tabelle 1b) wurden eingearbeitet (in M%): Clindamycin 1,2; DMSO 2,0; Ol. Menthae pip. 0,56; Ol. Cajeput 0,36; Ol. Salviae 0,24; Myrrhe 0,5; Extr. Chamomillae 1:1 2,0; Extr. Rad. Ratenhiae 1:1 2,0; 0,05 Saccharin.

### Anwendungsbeispiel 25

In das Basis-Gel 13 (vergl. Tabelle 2b) wurden eingearbeitet (in M%): 1,5 Hamamelis-Rinden-Extrakt 1:1; 1,5 Hamamelisblätter-Extrakt 1:1; 1,0 Kamillenbloten-Extrakt 1:1; Aescin 0,5; Rutin-tri-Hydrat 0,1; Polidocanol 3,0; Perubalsam 0,5; ätherische Öle: Sandelholz 0,6; Patchouli 0,6; Zypresse 0,6; Manuka 0,3; Niaouli 0,3; Neroli 0,3; Myrrhe 0,3.

### Anwendungsbeispiel 26

In das Basis-Gel 8 (vergl. Tabelle 1 b) wurden folgende Öle eingearbeitet (in M%): Campher 0,3; Niaouli 0,3; Myrte 0,4; Nelke 0,4; Cajeput 0,4; Manuka 0,4; Thymian 0,4; Lavendel 0,4.

**Anwendungsbeispiel 27**

In das Basis-Gel 20 (vergl. Tabelle 2b) wurden eingearbeitet (in M%): Calcium-Lactat 2,5; Ätherische Öle: Rosmarin 1,0, Palma rosa 1,0;Teebaum 1,0; Rosenholz 1,0; Bergbohnenkraut 1,0.

### Anwendungsbeispiel 28

Ein 74-jähriger Mann hat seit 2 Monaten Zahnbeschwerden, insbesondere an seinen 4 Brücken. Es handelt sich um parodontische Blutungen und Zahnhalsschmerzen. Der Patient wandte jeden Abend nach den Zähneputzen die in Anwendungsbeispiel 23 angegebene Zubereitung vorschriftsmäßig an. Nach 1 Woche waren die Beschwerden deutlich verringert und nach 14 Tagen völlig verschwunden.

### Anwendungsbeispiel 29

Eine 72-jährige Frau mit Engstellung der Zähne litt seit 3 Monaten unter ausgeprägter Parodontitis und Zahnfleischbluten. Zudem fand sich unten links eine lokale eitrig-entzündliche Taschenbildung. Die Patientin führte daraufhin regelmäßig abends nach der mechanischen Zahnreinigung vorschriftsmäßig die Behandlung mit der in Anwendungsbeispiel 24 angegebenen Zubereitung durch. Schon nach wenigen Tagen trat eine deutliche Besserung ein; nach 3 Wochen Behandlung war die Patientin beschwerdefrei, auch die lokale Entzündung war abgeklungen.

### Anwendungsbeispiel 30

Ein 63-jähriger Mann litt seit Jahren unter analen Beschwerden mit Schmerzen, Juckreiz und Blutungen (frisches Blut am Stuhl). Die spezielle Zubereitung (vergl. Formulierung von Anwendungsbeispiel 25) wurde 2x täglich morgens und abends angewendet. Bereits nach 14Tagen ließen Schmerz und Juckreiz deutlich nach und 4 Wochen später fanden sich auch keine Blutungen mehr.

### Anwendungsbeispiel 31

Eine 26-jährige Frau litt saisonal seit Jahren unter ausgeprägter infektiöser Rhinitis mit Begleit-Sinusitis, welche antibiotisch ohne Besserung austherapiert war. Mehrmals täglich wendete sie die in Anwendungsbeispiel 26 angegebene Zubereitung lokal-nasal an. Bereits nach wenigen Tagen reduzierten sich die Beschwerden entscheidend und nach 3 Wochen war die Patientin beschwerdefrei.

### Anwendungsbeispiel 32

Eine 32-jährige Frau litt seit 4 Monaten unter einer ausgeprägten Candida-Infektion mit bakterieller Über-Infektion durch E. choli ihrer Scheide, begleitet von Juckreiz und Brennen. Das Scheiden-Milieu erwies sich als alkalisch. Via Klistier wurden abendlich etwa 5ml der speziellen Zubereitung (vergl. Anwendungsbeispiel 27) appliziert. Schon nach 7 Tagen ließen die Beschwerden deutlich nach und 3 Wochen später war die Infektion ausgeheilt, wobei die Behandlungen nach 2 Wochen nur jeden 2. Tag erfolgten.

### Anwendungsbeispiel 33

In das Basis-Gel 20 (vergl. Tabelle 2b) wurden eingearbeitet (in M%):
Urea pura 5,0; Nachtkerzensamen-Öl 5,0; Glycyrrhitinsäure 1,0; Cardiospernum 0 5,0; Hamamelis 2,5; Johanniskraut 2,5; Ätherische Öle: Kamille blau 2,0; Geranie 1,5; Lavendel 2,5; Rosenholz 1,5; Sandelholz 0,8.

### Anwendungsbeispiel 34

Eine 18jährige Jugendliche litt seit mehreren Monaten unter unstillbarem starken Juckreiz an den Streckseiten der Unterarme, insbesondere der Ellenbogen, mit nächtlicher Selbstverletzung. Die Patientin wandte die Zubereitung des Anwendungsbeispiels 33 eine Woche lang jeden Abend unter Einreiben mit dem Gummifinger bis zum vollständigen Einziehen in die Haut an. 2 Wochen erfolgten dann die Behandlungen nur jeden 2. Tag. Bereits nach einer Woche war der Juckreiz soweit reduziert, dass die Patientin ungestört schlafen konnte und sich nicht mehr aufkratzte. Nach 3 Wochen waren auch die Entzündungen fast vollständig verschwunden.

## Patentansprüche

1. In situ gelbildende Zubereitung in Form einer W / O - Mikro-Emulsion, enthaltend:
- 30 bis 80 Gew.% einer Öl-Phase;
- 5 bis 45 Gew.% einer Mischung aus:
a) einem oder mehreren höher ethoxylierten nicht ionischen Gel- Emulgatoren der allgemeinen Formel I
R₁(ₘ₁) - Aₙ -R₂(ₘ₂), (I)
worin R₁, R₂ ausgewählt sind aus H, C₁₂ - C₂₂, gesättigten, und oder ungesättigten Fettsäureglycerid-Ölen, - C₁₂ - C₂₂, -Fettalkoholen, - C₁₂ - C_{22'}-Fettsäuren, oder Sorbitan- C₁₂-C₂₀ Fettsäureestem, (ggf.oligo/poly)Erythrol-Fett-säure-Estern, wobei A= (O - CH₂ -CH₂-), und n = 60 bis 1000 und m₁ = 0 bis 2, m₂ =1 bis 2 bedeuten, mit der Maßgabe, dass die Summe aus m₂ + m₂ (mit m₂, m2 ≠ H) ≤ 2 ist; wobei 0,005 bis 20 Gew.% des Gel-Emulgators in der Emulgator- Mischung vorhanden sind;
und
b) einem oder mehreren nicht ionischen Emulsionsemulgator der allgemeinen Formel II
{R₂)ₓ₁ - (A)_{y} -(R₁)ₓ₂ (II),
worin A= (O - CH₂ -CH₂-), oder (O - CH₂ -(CHₑ-) H-)- oder (O - B-R₂ₓ₁)- ; R₁, ausgewählt ist aus gesättigten und /oder ungesättigten - C₁₂ - C_{22'} Fettsäureglycerid-Ölen, - C₁₂ - C_{22'} -Fettalkoholen, - C₁₂ - C_{22'} -Fettsäuren, Sorbitan - C₁₂-C₂₀-Fettsäureestern; Sterolen, insbesondere natürlicher Herkunft; R₂ gewählt wird aus gesättigten, und /oder ungesättigten - C₁₂ - C_{22'}- Fettalkoholen, gesättigten und /oder ungesättigten C₁₂ - C_{22'} Fettsäureglycerid-Ölen, - C₁₂ - C_{22'} -Fettsäuren; H; mit y= 1-50 und mit x₁= 0 bis 10 und ,x₂ = 1 bis 5, wobei die Summe x₁ + x₂ (R_{1,2} ≠ H) 1-10 beträgt; und worin B (mono- oder poly)-Di- bis hexahydroxy- C₂ bis C₆- Alkohol, vorzugsweise (mono- oder poly)- Glycerol, - Ethylenglykol, -Erythrol, Sucrose oder - Sorbitol bedeutet;
- 0 bis 20 Gew.% eines oder mehrerer 1- oder 2-wertige C₂-C₈- Alkohole oder Mischungen hiervon;
- 1 bis 15 Gew.% Wasser oder wässerige Lösungen.

2. Zubereitung nach Anspruch 1, **dadurch gekennzeichnet, dass** weiterhin 0,001 bis 35 Gew.% lipophile, hydrophile, amphiphile Wirkstoffe oder Mischungen hiervon enthalten sind.

3. Zubereitung nach Anspruch 1, **dadurch gekennzeichnet, dass** weiterhin 0,1 bis 25 Gew.% Zusatzstoffe ausgewählt aus Penetrations- und Diffusionsverstärkern, Anti-Oxidantien, Salzen, pH-Wert- Regulatoren, Geschmacksstoffen, amphoteren Tensiden, Konservierungsstoffen, Wasserbindem oder Mischungen hiervon, enthalten sind.

4. Zubereitung nach einem der Ansprüche 1 bis 3, **dadurch gekennzeichnet, dass** die Menge an Lipoidphase 35 bis 70 Gew.% beträgt.

5. Zubereitung nach einem der Ansprüche 1 bis 4, **dadurch gekennzeichnet, dass** ein oder mehrere Substanzen, ausgewählt aus Estern aus monovalenten C₃-C₃₀- Alkoholen mit C₆-C₁₈- Carbonsäuren, ungesättigten und gesättigten C₁₄-C₂₂Fettsäuren, Di- C₁₂-C₂₂- Alkyl-Ethern und Triglyceriden gesättigter und ungesättigter C₁₄-C₂₀Fettsäuren, C₈-C₂₀ -Fett-Alkoholen als Komponenten der Öl-phase vorhanden sind.

6. Zubereitung nach einem der Ansprüche 1 bis 5, **dadurch gekennzeichnet, dass** das Verhältnis von Emulsions-Emulgator(en) und Gel-Emulgator(en) im Bereich 50:0,05 bis 50:20 beträgt.

7. Zubereitung nach einem der Ansprüche 1 bis 6, **dadurch gekennzeichnet, dass** der oder die Emulsions-Emulgatoren ausgewählt sind aus solchen der Formel II, worin A = (O - CH₂ -CH₂-), y, x₁ + x₂, R_{1,2} wie angegeben sind.

8. Zubereitung nach einem der Ansprüche 1 bis 7, **dadurch gekennzeichnet, dass** der oder die Emulsions- Emulgatoren ausgewählt sind aus:
- PEG (2-50)-mono-Estern, PEG (4-50)-di- C₁₂ - C_{22'} Estern, PEG(10-40) Glycerol C₁₂ - C₂₂ - Estern oder PEG (1-2)- Fettsäureethern gesättigter und ungesättigter C₁₂-C₂₀-Fettsäuren bzw. C₁₂-C₂₂-Fettalkoholen;
- PEG(4-50) -Glycerid Ölen mit x₁ + x₂ (R_{1,2} ≠ H) 1-4;
- PEG(4-50)-Sorbitan- C₁₂-C₂₀ -Fettsäuren (x₁ + x₂ (R_{1,2} ≠ H) 1-6) ;
- PEG(2-50)- C₁₂-C₁₆ Alkylethern;
- PEG(5-30)-Sterolen, mit Sterol ausgewählt aus Soja-Sterol, Phyto-Sterol, Cholesterol, Lano-Sterol, Sito-Sterol;
- Sucrose- C₁₂-C₂₂-Fettsäure-mono- und di-Ester, insbesondere Ester mit Stearat, Palmitat, Laurat;
- Propylenglykol-mono-und di-Ester der C₈ -C₂₂-Fettsäuren, wobei Laurat, Myristat, Stearat, Ricinoleat und Oleat bevorzugt sind;
oder Mischungen hiervon.

9. Zubereitung nach einem der Ansprüche 1 bis 8, **dadurch gekennzeichnet, dass** der oder die ethoxylierten Gel- Emulgatoren ausgewählt sind aus:
- PEG (60-1000)-mono-Estern oder PEG (400)- Di-Estern von C₁₄-C₂₀ -Fettsäuren wie PEG(60-1000)- Mono-Oleat, -Stearat, -Laurat, -Ricinolat, PEG (400) di- Oleat, -di-Stearat;
- natürlichen und hydrierten natürlichen PEG(60 bis 200)- Öl-Emulgatoren wie PEG (60-200) Castoroil, PEG (60-200) Castoroil(hydriert),PEG(60-200) - Maisöl, -Olivenöl, -Erdnussöl, -Palmkernöl, -Mandelöl, -Aprikosenkernöl;
- PEG(60; 80)-Sorbitan- C₁₄-C₂₀ -Fettsäurestem wie PEG(60; 80)-Sorbitan-Laurat, -Palmitat, -Stearat, -Oleat;
- PEG(n)-penta-Erythryl-tetra-Stearat mit n= 150;
- PEG(60-400)- C₁₂-C₁₈ -Alkyl-Ethern wie PEG(60-400)- Oleyl, Stearyl, Lauryl, Cetylether
oder Mischungen hiervon.

10. Zubereitung nach einem der Ansprüche 1 bis 9, **dadurch gekennzeichnet, dass** der C₁₂-C₂₀ Fettsäure- /Fettalkohol- bzw. C₁₂-C₂₀ -Alkylteil der Emulgatoren abgeleitet ist von Ölsäure, Linolensäure, Laurinsäure, Linolsäure, Palmitiinsäure, Caprinsäure, Ricinolsäure, Stearinsäure, Cetylalkohol, Laurylalkohol, Stearylalkohol, Oleylalkohol.

11. Zubereitung nach einem der Ansprüche 1 bis 10, **dadurch gekennzeichnet, dass** ein oder mehrere Wirkstoffe enthalten sind, ausgewählt aus Pharmaka, Pflanzeninhaltsstoffen, oder Mischungen hiervon.

12. Zubereitung nach Anspruch 11, **dadurch gekennzeichnet, dass** ein oder mehrere Wirkstoffe enthalten sind, ausgewählt aus a) anti-bakteriellen, antiinfektiösen, anti-septischen, b)adstringierenden, c) analgetischen, d) anästhesierenden, e) antihämorrhagischen, phlebotonischen, f) anti-histaminischen, anti-allergischen, g) anti-koagulierenden, h) anti-mykotischen, i) anti-ödematösen, anti-koagulierenden, j)anti-parasitischen, k) anti-phlogistischen, I)anti-spastischen, relaxierenden; m) anti-viralen, n) regenerierenden, wundheilenden, pflegenden, o) hyperämisierenden, p) immun-modulatorischen, q) juckreizstillenden, r)vasodilatorischen, s) anti-oxidativen, t) befeuchtenden Wirkstoffen, v) Sympathomimetika, w) Parasympathomimetika; x) Carboanhydrase-Hemmern; y) Prostaglanin-Derivaten,z) Kontrazeptiva; oder Mischungen hiervon.

13. Zubereitung nach einem der Ansprüche 1 bis 12, **dadurch gekennzeichnet, dass** ein oder mehrere Wirkstoffe enthalten sind, ausgewählt aus Kamillenöl, Kamillenblätterextrakt, Rosenholzöl, Sandelholzöl, Rosmarinöl, Palma rosa Öl, Myrrhe, Lavendelöl, Hammamelisblätter- Extrakt, Teebaumöl, Bergbohnenkraut, Thymianöl, Vitamin A,B, C, D, E, H, oder Mischungen hiervon.

14. Zubereitung nach einem der Ansprüche 1 bis 13, **dadurch gekennzeichnet, dass** 1 bis 15 Gew-% eines 1-und / oder 2-wertigen Alkohols, ausgewählt aus Ethanol, Isopropanol, Propylenglykol; Hexandiolen, Oktandiolen oder Mischungen hiervon vorhanden ist.

15. Zubereitung nach einem der Ansprüche 1 bis 14, **dadurch gekennzeichnet, dass** als Öl(e)(Lipoide) ein oder mehrere Substanzen, ausgewählt aus Estern aus monovalenten C₃-C₃₀- Alkoholen mit C₆-C₁₈- Carbonsäuren; gesättigten oder ungesättigten C₁₂-C₂₀-Fettsäuren natürlicher Herkunft: Glycerolestern oder C₁₂-C₂₀- Alkylethem gesättigter und ungesättigter C₁₂-C₂₀-fettsäuren natürlicher und synthetischer Herkunft; oder Mischungen hiervon, enthalten sind.

16. Zubereitung nach einem der Ansprüche 1 bis 15 zur topischen kosmetischen-Anwendung auf Haut, befeuchteter Haut, oder Schleimhaut.

17. Zubereitung nach einem der Ansprüche 1-15 zur topischen dermatologischen Anwendung auf Haut, befeuchtete Haut, Schleimhaut.

18. Zubereitung nach einem der Ansprüche 1 bis 15 zur Verwendung bei der Behandlung oder Prophylaxe entzündungsbedingter, hormoneller, infektionsbedingter Störungen der Schleimhaut des Mund-Rachen-Raumes, des Analbereichs, des Urogenitaltrakts, insbesondere der Scheide, sowie Erkrankungen des Auges.

19. Verwendung einer Zubereitung nach einem der Ansprüche 1 bis 15 zur Herstellung eines Mittels zur Behandlung oder Prophylaxe entzündungsbedingter, hormoneller, infektionsbedingter Störungen der Schleimhaut des Mund-Rachen-Raumes, des Analbereichs, des Urogenitaltrakts, insbesondere der Scheide, sowie Erkrankungen des Auges.

20. Verwendung nach Anspruch 19 zur Behandlung von Gingivitis, Stomatitis aphtosa et ulcerosa, Rhagaden, Soor, Herpes, Parodontose, Parodontitis, Pharyngitis simplex et sicca, Laryngitis, Glossitis, Kariesprophylaxe, Infekten nach Zahn-Extraktionen und nach oral-chirurgischen Eingriffen, Prothesen-Druckstellen, Plaques, Foetor ex ore, (im Mundraum); Rhinitis, Sinusitis, Pollinosis im Nasenraum ; Thrombophlebitis, Proctitis, Rhagaden, Fissuren, Pruritus, Brennen, Schmerzen, Blutungen und Ekzeme im Analbereich; Fluor vaginalis, Colpitis, Cervizitis, atrophische Entzündungen, Pelvipathie, Ostrogen-Mangel mit Genital-Atropien, lokale Kontrazeptiva sowie Zubereitungen zur Restitution des Scheiden-Milieus (im Scheidenbereich); Entzündungen, Infekten der Bindehaut oder Wundbehandlung derartiger Schleimhautgewebe wie auch Erkrankungen des Auges oder Neurodermitis.

21. Zubereitung nach einem der Ansprüche 1-18 zur topischen Anwendung durch manuelles Auftragen, Einreiben und / oder Sprühen.

## Claims

1. In situ gel-forming preparation in the form of a W/O microemulsion containing:
- 30 to 80 wt.% of an oil phase;
- 5 to 45 wt.% of a mixture of:
a) one or more highly ethoxylated nonionic gel emulsifiers of the general formula I
R₁(m₁)-Aₙ-R₂(m₂) (I),
in which R₁, R₂ are selected from H, -C₁₂-C_{22'} saturated and/or unsaturated fatty acid glyceride oils, -C₁₂-C_{22'} fatty alcohols, -C₁₂-C_{22'} fatty acids, or sorbitan C₁₂-C₂₀ fatty acid esters, (optionally oligo/poly)erythrol fatty acid esters, wherein A = (O-CH₂-CH₂), and n = 60 to 1000 and m₁ = 0 to 2, m₂ = 1 to 2, with the proviso that the sum of m₂ + m₂ (with m₂, m₂ ≠ H) is ≤ 2; wherein 0.005 to 20 wt.% of the gel emulsifier are present in the emulsifier mixture;
and
b) one or more nonionic emulsion emulsifiers of the general formula II
(R₂)ₓ₁-(A)_{y}-(R₁)ₓ₂ (II),
in which A = (O-CH₂-CH₂-) or (O-CH₂-(CH₃-)H-)- or (O-B-R₂ₓ₁)-; R₁ is selected from among saturated and/or unsaturated -C₁₂-C_{22'} fatty acid glyceride oils, -C₁₂-C_{22'} fatty alcohols, -C₁₂-C_{22'} fatty acids, sorbitan C₁₂-C₂₀-fatty acid esters; sterols, in particular of natural origin; R₂ is selected from saturated and/or unsaturated -C₁₂-C₂₂ fatty alcohols, saturated and/or unsaturated C₁₂-C_{22'} fatty acid glyceride oils, -C₁₂-_{22'} fatty acids; H; with y = 1-50 and with x₁ = 0 to 10 and x₂ = 1 to 5, wherein the sum x1 + x2 (R_{1, 2} ≠ H) amounts to 1-10; and in which B means (mono- or poly)-di-to hexahydro-C₂ to C₆ alcohol, preferably (mono- or poly)-glycerol, -ethylene glycol, -erythrol, sucrose or -sorbitol;
- 0 to 20 wt.% of one or more mono- or dihydric C₂-C₈ alcohols or mixtures thereof;
- 1 to 15 wt.% of water or aqueous solutions.

2. A preparation according to claim 1, **characterised in that** it furthermore contains 0.001 to 35 wt.% of lipophilic, hydrophilic, amphiphilic active ingredients or mixtures thereof.

3. A preparation according to claim 1, **characterised in that** it furthermore contains 0.1 to 25 wt.% of additives selected from penetration and diffusion promoters, antioxidants, salts, pH value regulators, flavours, amphoteric surfactants, preservatives, water-binding agents or mixtures thereof.

4. A preparation according to any one of claims 1 to 3, **characterised in that** the quantity of lipoid phase amounts to 35 to 70 wt.%.

5. A preparation according to any one of claims 1 to 4, **characterised in that** one or more substances selected from esters of monovalent C₃-C₃₀ alcohols with C₆-C₁₈ carboxylic acids, unsaturated and saturated C₁₄-C₂₂ fatty acids, di-C₁₂-C₂₂-alkyl ethers and triglycerides of saturated and unsaturated C₁₄-C₂₀ fatty acids, C₈-C₂₀ fatty alcohols are present as components of the oil phase.

6. A preparation according to any one of claims 1 to 5, **characterised in that** the ratio of emulsion emulsifier(s) and gel emulsifier(s) is in the range 50:0.05 to 50:20.

7. A preparation according to any one of claims 1 to 6, **characterised in that** the emulsion emulsifier(s) is/are selected from those of formula II in which A = (O-CH₂-CH₂-), y, x₁ + x₂, R_{1, 2} as stated.

8. A preparation according to any one of claims 1 to 7, **characterised in that** the emulsion emulsifier(s) is/are selected from:
- PEG (2-50) monoesters, PEG (4-50) di-C₁₂-C_{22'}-esters, PEG (10-40) glycerol C₁₂-C₂₂ esters or PEG (1-2) fatty acid ethers of saturated and unsaturated C₁₂-C₂₀ fatty acids or C₁₂-C₂₂ fatty alcohols;
- PEG (4-50) glyceride oils with x₁ + x₂ (R_{1, 2} ≠ H) 1-4;
- PEG (4-50) sorbitan C₁₂-C₂₀ fatty acids (x₁ + x₂ = (R_{1, 2} ≠ H) 1-6);
- PEG (2-50) C₁₂-C₁₆ alkyl ethers;
- PEG (5-30) sterols, with sterol selected from soya sterol, phytosterol, cholesterol, lanosterol, sitosterol;
- sucrose C₁₂-C₂₂ fatty acid mono- and diesters, in particular esters with stearate, palmitate, laurate;
- propylene glycol mono- and diesters of C₈-C₂₂ fatty acids, with laurate, myristate, stearate, ricinoleate and oleate being preferred;
or mixtures thereof.

9. A preparation according to any one of claims 1 to 8, **characterised in that** the ethoxylated gel emulsifier(s) is/are selected from:
- PEG (60-1000) monoesters or PEG (400) diesters of C₁₄-C₂₀ fatty acids such as PEG (60-1000) monooleate, monostearate, monolaurate, monoricinoleate, PEG (400) dioleate, distearate;
- natural and hydrogenated natural PEG (60 to 200) oil emulsifiers such as PEG (60-200) castor oil, PEG (60-200) castor oil (hydrogenated), PEG (60-200) corn oil, olive oil, peanut oil, palm kernel oil, almond oil, apricot kernel oil;
- PEG (60-80) sorbitan C₁₄-C₂₀ fatty acid esters such as PEG (60-80) sorbitan laurate, palmitate, stearate, oleate;
- PEG (n) pentaerythryl tetrastearate with n = 150;
- PEG (60-400) C₁₂-C₁₈ alkyl ethers such as PEG (60-400) oleyl, stearyl, lauryl, cetyl ether
or mixtures thereof.

10. A preparation according to any one of claims 1 to 9, **characterised in that** the C₁₂-C₂₀ fatty acid/fatty alcohol or C₁₂-C₂₀ alkyl moiety of the emulsifiers is derived from oleic acid, linolenic acid, lauric acid, linoleic acid, palmitic acid, capric acid, ricinoleic acid, stearic acid, cetyl alcohol, lauryl alcohol, stearyl alcohol, oleyl alcohol.

11. A preparation according to any one of claims 1 to 10, **characterised in that** it contains one or more active ingredients selected from pharmaceuticals, plant constituents, or mixtures thereof.

12. A preparation according to claim 11, **characterised in that** it contains one or more active ingredients selected from a) antibacterial, antiinfective, antiseptic, b) astringent, c) analgesic, d) anaesthetic, e) antihaemorrhagic, phlebotonic, f) antihistaminic, antiallergic, g) anticoagulant, h) antimycotic, i) antioedoematous, anticoagulant, j) antiparasitic, k) antiinflammatory, I) antispasmodic, relaxant; m) antiviral, n) regenerative, wound-healing, conditioning, o) circulation-boosting, p) immunomodulatory, q) itch-relieving, r) vasodilatory, s) antioxidant, t) moistening active ingredients, v) sympathomimetics, w) parasympathomimetics; x) carboanhydrase inhibitors; y) prostaglandin derivatives, z) contraceptives; or mixtures thereof.

13. A preparation according to any one of claims 1 to 12, **characterised in that** it contains one or more active ingredients selected from chamomile oil, chamomile leaf extract, rosewood oil, sandalwood oil, rosemary oil, palmarosa oil, myrrh, lavender oil, witchhazel leaf extract, tea tree oil, winter savory, thyme oil, vitamin A, B, C, D, E, H, or mixtures thereof.

14. A preparation according to any one of claims 1 to 13, **characterised in that** 1 to 15 wt.% of a mono- and/or dihydric alcohol, selected from ethanol, isopropanol, propylene glycol; hexanediols, octanediols or mixtures thereof, is present.

15. A preparation according to any one of claims 1 to 14, **characterised in that**, as oil(s) (lipoids), it contains one or more substances selected from esters of monovalent C₃-C₃₀ alcohols with C₆-C₁₈ carboxylic acids; saturated or unsaturated C₁₂-C₂₀ fatty acids of natural origin; glycerol esters or C₁₂-C₂₀ alkyl ethers of saturated and unsaturated C₁₂-C₂₀ fatty acids of natural and synthetic origin; or mixtures thereof.

16. A preparation according to any one of claims 1 to 15 for topical cosmetic use on skin, moistened skin or mucosa.

17. A preparation according to any one of claims 1-15 for topical dermatological use on skin, moistened skin, mucosa.

18. A preparation according to any one of claims 1 to 15 for use in the treatment or prevention of inflammatory, hormonal, infectious disorders of the mucosa of the oral cavity or pharynx, of the anal region, of the urogenital tract, in particular of the vagina, and of diseases of the eye.

19. Use of a preparation according to any one of claims 1 to 15 for producing an agent for the treatment or prevention of inflammatory, hormonal, infectious disorders of the mucosa of the oral cavity or pharynx, of the anal region, of the urogenital tract, in particular of the vagina, and of diseases of the eye.

20. Use according to claim 19 for the treatment of gingivitis, stomatitis aphtosa and ulcerative stomatitis, rhagades, thrush, herpes, periodontosis, periodontitis, pharyngitis simplex and pharyngitis sicca, laryngitis, glossitis, prevention of caries, infections after tooth extraction and after oral surgery, prosthesis pressure sores, plaques, halitosis (in the oral cavity); rhinitis, sinusitis, nasal pollinosis; thrombophlebitis, proctitis, rhagades, fissures, pruritus, stinging, pain, bleeding and eczema in the anal region; vaginal discharge, colpitis, cervicitis, atrophic inflammation, chronic pelvic pain, oestrogen deficiency with genital atrophy, local contraceptives together with preparations for restoring the vaginal environment (in the vaginal region); inflammation, infections of the conjunctiva or wound treatment of such mucosal tissue as well as diseases of the eye or neurodermatitis.

21. A preparation according to any one of claims 1-18 for topical application by manual application, rubbing in and/or spraying.

## Revendications

1. Préparation formant in situ un gel sous forme d'une microémulsion E/H, contenant :
- 30 à 80% en poids d'une phase huileuse ;
- 5 à 45% en poids d'un mélange constitué par :
a) un ou plusieurs émulsifiants de gel non ioniques, hautement éthoxylés de formule générale I
R₁(ₘ₁)-Aₙ-R₂(ₘ₂), (I)
R₁, R₂ étant choisis parmi H, les huiles de glycérides d'acides gras en C₁₂-C₂₂ saturés et/ou insaturés ; les alcools gras en C₁₂-C₂₂ ; les acides gras en C₁₂-C₂₂; ou les esters de sorbitane d'acides gras en C₁₂-C₂₀ ; les esters de (le cas échéant oligo ou poly)érythrol d'acides gras, A = (O-CH₂-CH₂-), et n = 60 à 1000 et m₁ = 0 à 2, m₂ = 1 à 2, à condition que la somme de m₁ + m₂ (avec R₁, R₂ ≠ H) ≤ 2 ; le mélange d'émulsifiants contenant 0,005 à 20% en poids de l'émulsifiant de gel ; et
b) un ou plusieurs émulsifiants d'émulsion non ioniques de formule générale II
(R₂)ₓₗ-(A)_{y}-(R₁)ₓ₂ (II),
A=(O-CH₂-CH₂-), ou (O-CH₂-(CH₃-)H-)- ou (O-B-R₂ₓ₁)- ; R₁ étant choisi parmi les huiles de glycérides d'acides gras en C₁₂-C₂₂ saturés et/ou insaturés, les alcools gras en C₁₂-C₂₂, les acides gras en C₁₂-C₂₂, les esters de sorbitane d'acides gras en C₁₂-C₂₀, les stérols, en particulier d'origine naturelle ; R₂ étant choisi parmi les alcools gras en C₁₂-C₂₂ saturés et/ou insaturés, les huiles de glycérides d'acides gras en C₁₂-C₂₂ saturés et/ou insaturés, les acides gras en C₁₂-C₂₂, H ; y = 1-50 et x₁ = 0 à 10 et x₂ = 1 à 5, la somme x₁ + x₂ (R_{1,2} ≠ H) valant 1-10 ; et B signifiant un monoalcool ou un polyalcool en C₂ à C₆ à fonction di-à-hexahydroxy, de préférence le monoglycérol ou le polyglycérol, le monoéthylèneglycol ou le polyéthylèneglycol, le monoérythrol ou le polyérythrol, le sucrose ou le monosorbitol ou le polysorbitol ;
- 0 à 20% en poids d'un ou de plusieurs alcools en C₂-C₈ monovalents ou divalents ou leurs mélanges ;
- 1 à 15% en poids d'eau ou de solutions aqueuses.

2. Composition selon la revendication 1, **caractérisée en ce qu'**elle contient en outre 0,001 à 35% en poids de substances actives lipophiles, hydrophiles, amphiphiles ou leurs mélanges.

3. Composition selon la revendication 1, **caractérisée en ce qu'**elle contient en outre 0,1 à 25% en poids d'additifs choisis parmi les agents de renforcement de la pénétration et de la diffusion, les antioxydants, les sels, les régulateurs du pH, les agents gustatifs, les agents tensioactifs amphotères, les conservateurs, les liants d'eau ou leurs mélanges.

4. Composition selon l'une quelconque des revendications 1 à 3, **caractérisée en ce que** la quantité de phase lipoïde représente 35 à 70% en poids.

5. Composition selon l'une quelconque des revendications 1 à 4, **caractérisée en ce qu'**elle contient une ou plusieurs substances, choisies parmi les esters d'alcools en C₃-C₃₀ monovalents avec des acides carboxyliques en C₆-C₁₈, les acides gras en C₁₄-C₂₂ saturés et insaturés, les di-C₁₂-C₂₂-alkyléthers et les triglycérides d'acides gras en C₁₄-C₂₀ saturés et insaturés, les alcools gras en C₈-C₂₀ comme composants de la phase huileuse.

6. Composition selon l'une quelconque des revendications 1 à 5, **caractérisée en ce que** le rapport d'émulsifiant(s) d'émulsion et d'émulsifiant(s) de gel se situe dans la plage de 50:0,05 à 50:20.

7. Composition selon l'une quelconque des revendications 1 à 6, **caractérisée en ce que** le ou les émulsifiants d'émulsion sont choisis parmi ceux de formule II, A = (O-CH₂-CH₂-), y, x₁ + x₂, R_{1,2} étant comme indiqués.

8. Composition selon l'une quelconque des revendications 1 à 7, **caractérisée en ce que** le ou les émulsifiants d'émulsion sont choisis parmi :
- les PEG (2-50)-mono-esters, les PEG (4-50)-di-C₁₂-C₂₂-esters, les PEG(10-40) glycérol C₁₂-C₂₂-esters ou les PEG (1-2)-éthers d'acides gras, d'acides gras en C₁₂-C₂₀ ou, selon le cas, d'alcools gras en C₁₂-C₂₂ saturés ou insaturés ;
- les huiles de PEG(4-50)-glycérides avec x₁ + x₂ (R_{1,2} ≠ H) 1-4 ;
- les PEG(4-50)-sorbitane-acides gras en C₁₂-C₂₀ (x₁ + x₂ (R_{1,2} ≠ H) 1-6) ;
- les PEG(2-50)-C₁₂-C₁₆-alkyléthers ;
- les PEG(5-30)-stérols, le stérol étant choisi parmi le stérol de soja, le phytostérol, le cholestérol, le lanostérol, le sitostérol ;
- les monoesters et les diesters de saccharose-acides gras en C₁₂-C₂₂, en particulier les esters avec du stéarate, du palmitate, du laurate ;
- les propylèneglycolmonoesters et les propylèneglycoldiesters des acides gras en C₈-C₂₂, le laurate, le myristate, le stéarate, le ricinoléate et l'oléate étant préférés ;
ou leurs mélanges.

9. Composition selon l'une quelconque des revendications 1 à 8, **caractérisée en ce que** le ou les émulsifiants de gel sont choisis parmi :
- les PEG (60-1000)-mono-esters ou les PEG (400)-diesters d'acides gras en C₁₄-C₂₀ tels que le monooléate, le monostéarate, le monolaurate, le monoricinoléate de PEG(60-1000), le dioléate, le distéarate de PEG (400);
- les PEG-huiles naturelles et naturelles hydrogénées, telles que la PEG (60-200)-huile de ricin, la PEG (60-200)-huile de ricin hydrogénée, la PEG(60-200)-huile de maïs, la PEG(60-200)-huile d'olive, la PEG(60-200)-huile d'arachide, la PEG(60-200)-huile de noyau de palme, la PEG(60-200)-huile d'amande, la PEG(60-200)-huile de noyau d'abricot ;
- les esters de PEG(60 ; 80)-sorbitane d'acides gras en C₁₄-C₂₀, tels que le laurate, le palmitate, le stéarate, l'oléate de PEG(60 ; 80)-sorbitane ;
- le tétrastéarate de PEG(n)-pentaéythryle, n = 150 ;
- les PEG(60-400)-C₁₂-C₁₈-alkyléthers tels que le PEG(60-400)-oléyléther, le PEG(60-400)-stéaryléther, le PEG(60-400)-lauryléther, le PEG(60-400)-cétyléther
ou leurs mélanges.

10. Composition selon l'une quelconque des revendications 1 à 9, **caractérisée en ce que** la partie d'acide gras/alcool gras en C₁₂-C₂₀ ou, selon le cas, la partie alkyle en C₁₂-C₂₀ des émulsifiants est dérivée de l'acide oléique, linolénique, laurique, linoléique, palmitique, caprique, ricinoléique, stéarique, de l'alcool cétylique, de l'alcool laurylique, de l'alcool stéarylique, de l'alcool oléylique.

11. Composition selon l'une quelconque des revendications 1 à 10, **caractérisée en ce qu'**elle contient une ou plusieurs substances actives, choisies parmi les produits pharmaceutiques, les constituants végétaux ou leurs mélanges.

12. Composition selon la revendication 11, **caractérisée en ce qu'**elle contient une ou plusieurs substances actives, choisies parmi les substances actives a) antibactériennes, anti-infectieuses, antiseptiques, b) astringentes, c) analgésiques, d) anesthésiantes, e) antihémorragiques, phlébotoniques, f) antihistaminiques, antiallergiques, g) anticoagulantes, h) antimycosiques, i) anti-oedémateuses, anticoagulantes, j) antiparasitiques, k) antiphlogistiques, I) antispastiques, relaxantes ; m) antivirales, n) régénératrices, cicatrisantes, de soin, o) hyperémiantes, p) immunomodulatrices, q) antiprurigineuses, r) vasodilatatrices, s) antioxydantes , t) hydratantes, v) les sympathomimétiques, w) les parasympathomimétiques ; x) les inhibiteurs de la carboanhydrase ; y) les dérivés de la prostaglandine, z) les contraceptifs ; ou leurs mélanges.

13. Composition selon l'une quelconque des revendications 1 à 12, **caractérisée en ce qu'**elle contient une ou plusieurs substances actives, choisies parmi l'huile de camomille, l'extrait de feuilles de camomille, l'huile de bois de rose, l'huile de bois de santal, l'huile de romarin, l'huile de Palma rosa, la myrrhe, l'huile de lavande, l'extrait de feuilles d'hamamélis, l'huile de l'arbre à thé, la sarriette des montagnes, l'huile de thym, la vitamine A, B, C, D, E, H, ou leurs mélanges.

14. Composition selon l'une quelconque des revendications 1 à 13, **caractérisée en ce qu'**elle contient 1 à 15% en poids d'un alcool monovalent et/ou divalent, choisi parmi l'éthanol, l'isopropanol, le propylèneglycol ; les hexanediols, les octanediols ou leurs mélanges.

15. Composition selon l'une quelconque des revendications 1 à 14, **caractérisée en ce qu'**elle contient comme huile(s) (lipoïde(s)) une ou plusieurs substances choisies parmi les esters d'alcools en C₃-C₃₀ monovalents avec des acides carboxyliques en C₆-C₁₈ ; les acides gras en C₁₂-C₂₀ saturés ou insaturés d'origine naturelle ; les esters de glycérol ou les C₁₂-C₂₀-alkyléthers d'acides gras en C₁₂-C₂₀ saturés ou insaturés d'origine naturelle et synthétique ; ou leurs mélanges.

16. Composition selon l'une quelconque des revendications 1 à 15 pour une utilisation cosmétique topique sur la peau, les cheveux humides ou les muqueuses.

17. Composition selon l'une quelconque des revendications 1-15 pour une utilisation dermatologique topique sur la peau, les cheveux humides, les muqueuses.

18. Composition selon l'une quelconque des revendications 1 à 15 pour une utilisation lors du traitement ou de la prophylaxie de troubles provoqués par une inflammation, hormonaux, provoqués par une infection de la muqueuse de l'espace oro-pharyngien, de la zone anale, du tractus uro-génital, en particulier du vagin, ainsi que de maladies des yeux.

19. Utilisation d'une composition selon l'une quelconque des revendications 1 à 15 pour la préparation d'un agent destiné au traitement ou à la prophylaxie de troubles provoqués par une inflammation, hormonaux, provoqués par une infection de la muqueuse de l'espace oro-pharyngien, de la zone anale, du tractus uro-génital, en particulier du vagin, ainsi que les maladies de yeux.

20. Utilisation selon la revendication 19 pour traiter une gingivite, la stomatite aphteuse et ulcéreuse, les rhagades, le muguet, l'herpes, une parodontose, une parodontite, une pharyngite simple et sèche, une laryngite, une glossite, pour la prophylaxie des caries, pour traiter les infections après des extractions dentaires et après des interventions chirurgicales orales, les endroits de compression de prothèses, la plaque, le Foetor ex ore, (dans l'espace buccal) ; la rhinite, la sinusite, la pollinose dans l'espace nasal ; la thrombophlébite, la proctite, les rhagades, les fissures, le prurit, les brûlures, les douleurs, les saignements et l'eczéma dans la zone anale ; les pertes vaginales, la colpite, la cervicite, les inflammations atrophiques, la pelvipathie, les troubles oestrogéniques avec des atrophies génitales, les contraceptifs oraux ainsi que les compositions pour la restitution du milieu vaginal (dans la vagin) ; les inflammations, les infections de la conjonctive, ou le traitement de plaies de tissus des muqueuses ainsi que les maladies des yeux ou la neurodermite.

21. Composition selon l'une quelconque des revendications 1-18 pour une utilisation topique par application manuelle, frottement et/ou pulvérisation.
